# EUROPEAN PATENT APPLICATION

(11) **EP 1 913 877 A1**
(43) Date of publication of application: **23.04.2008**
(21) Application number: 05780221.7
(22) Date of filing: 12.08.2005
(51) Int. Cl.: A61B 10/00, A61B 1/00

(54) **ENDOSCOPE ACCESSORY, BIOTISSUE ANALYTICAL PROCESSING SYSTEM AND METHOD OF SAMPLING FOR TISSUE ANALYTICAL PROCESSING**

(71) Applicant: Olympus Corporation, Tokyo 151-0072 (JP)
(72) Inventor: OAKI, Yoshina c/o Intellectual Property Support Department, Hachioji-shi Tokyo 192-8512 (JP); KARAKI, Sachiko c/o Intellectual Property Support Department, Hachioji-shi Tokyo 192-8512 (JP); OTANI, Yutaka c/o Intellectual Property Support Department, Hachioji-shi Tokyo 192-8512 (JP); KOJIMA, Kiyotsugu c/o Intellectual Property Support Department, Hachioji-shi Tokyo 192-8512 (JP); SAITO, Tatsuya c/o Intellectual Property Support Department, Hachioji-shi Tokyo 192-8512 (JP); NAKAMURA, Toshio c/o Intellectual Property Support Department, Hachioji-shi Tokyo 192-8512 (JP); ISHIGURO, Tsutomu c/o Intellectual Property Support Department, Hachioji-shi Tokyo 192-8512 (JP); MANABE, Sugio c/o Intellectual Property Support Department, Hachioji-shi Tokyo 192-8512 (JP); TAKAMURA, Koji c/o Intellectual Property Support Department, Hachioji-shi Tokyo 192-8512 (JP); SAKAMOTO, Hiroko c/o Intellectual Property Support Department, Hachioji-shi Tokyo 192-8512 (JP)
(74) Representative: von Hellfeld, Axel
(86) International application number: PCT/JP2005/014830
(87) International publication number: WO 2007/020680

(57) **Abstract**

An endoscopic therapeutic device (1) includes an elongated insertion section (15) which is inserted into a living body through a therapeutic device channel (10) of an endoscope (2), a tubular puncture needle (16) which is disposed at a distal end portion of the insertion section (15) and is made to pierce a living body tissue, tissue-sampling means (18) for sampling the living body tissue in a state in which the puncture needle (16) pierces the living body tissue, and tissue-processing means (22) for subjecting the living body tissue, which is collected by the tissue-sampling means (18), to a process for analysis.

## Description

### Technical Field

The present invention relates to an endoscopic therapeutic device which is inserted into a living body through a therapeutic device channel of an endoscope and is used in combination with the endoscope, a living body tissue analyzing and processing system, and a sample-taking method for a tissue analysis process.

### Background Art

In general, a living body tissue in a living body is collected for biomolecular analysis. In this kind of analysis process of a living body tissue, a device disclosed in, e.g. Jpn. Pat. Appln. KOKAI Publication No. 2001-275947 (Patent Document 1) has been conventionally used. In this case, use is made of an endoscopic puncture needle which is inserted into the living body through a therapeutic device channel of an endoscope for observing the inside of the living body. The endoscopic puncture needle is inserted into the living body under the observation by the endoscope and is pierced into a target living body tissue, and in this state the living body tissue is collected. The collected living body tissue is transferred to a place for an inspection, which is different from an endoscopic inspection room, and an inspection, such as pathologic diagnosis, is conducted.

### Disclosure of Invention

In the apparatus of Patent Document 1, a length of time is needed from the sampling of the living body tissue to the diagnosis. Thus, there is a concern that the condition of the living body tissue may deteriorate, and it is difficult to conduct exact diagnosis.

The present invention has been made in consideration of the above-described circumstances, and the object of the invention is to provide an endoscopic therapeutic device, a living body tissue analyzing and processing system, and a sample-taking method for a tissue analysis process, which can make it possible to keep a collected living body tissue in a fresh state and to perform exact diagnosis.

According to a first aspect of the present invention, an endoscopic therapeutic device comprising: an elongated insertion section which is inserted into a living body through a therapeutic device channel of an endoscope which observes an inside of the living body; a tubular puncture needle which is disposed at a distal end portion of the insertion section and is made to pierce a living body tissue; tissue-sampling means for sampling the living body tissue in a state in which the puncture needle pierces the living body tissue; and tissue-processing means for subjecting the living body tissue, which is collected by the tissue-sampling means, to a process for analysis.

According to the above-described aspect, the tubular puncture needle, which is disposed at the distal end portion of the elongated insertion section that is inserted into the living body through the therapeutic device channel of the endoscope which observes the inside of the living body, is made to pierce the living body tissue. The living body tissue is collected by the tissue-sampling means in the state in which the puncture needle pierces the living body tissue. Then, the collected living body tissue is subjected to the process for analysis by the tissue-processing means.

Preferably, the insertion section includes an elongated tube body, and the puncture needle is formed at a distal end portion of the tube body, the tissue-sampling means comprises a syringe which includes an outer cylinder member which is detachably coupled to a proximal end portion of the tube body, and a shaft-shaped piston member which is axially slidably inserted in a cylinder of the outer cylinder member, the living body tissue being collected into an inside of the outer cylinder member through the puncture needle and the tube body by a suction operation in which the piston member is slid in an outward direction of the outer cylinder member, and the tissue-processing means includes a freezing device which freezes the living body tissue which is collected in the syringe.

In the above-described structure, the outer cylinder member of the tissue-sampling means that is the syringe is detachably coupled to the proximal end portion of the elongated tube body which has the puncture needle at the distal end portion thereof. The shaft-shaped piston member is axially slidably inserted in the cylinder of the outer cylinder member. Further, the living body tissue is collected into the inside of the outer cylinder member through the puncture needle and the tube body by the suction operation in which the piston member is slid in the outward direction of the outer cylinder member. Subsequently, the living body tissue which is collected in the syringe is frozen by the freezing device of the tissue-processing means.

Preferably, the freezing device includes a Peltier element which is mounted in the outer cylinder member of the syringe, and a power supply device which supplies power to the Peltier element.

In the above-described structure, power is supplied to the Peltier element from the power supply device. Thereby, the outer cylinder member of the syringe is cooled by the Peltier element, and the living body tissue that is collected in the syringe is frozen.

Preferably, the insertion section includes an electrically conductive elongated tube body, and the puncture needle is formed at a distal end portion of the tube body, the tissue-sampling means comprises a syringe, which includes an outer cylinder member that is detachably coupled to a proximal end portion of the tube body and a shaft-shaped piston member that is axially slidably inserted in a cylinder of the outer cylinder member, and a tissue-sampling section which is disposed at the distal end portion of the tube body, and the tissue-sampling means includes a tissue-sampling mechanism which samples the living body tissue into an inside of the tissue-sampling section through the puncture needle by a suction operation in which the piston member is slid in an outward direction of the outer cylinder member, and the tissue-processing means includes a freezing device which freezes the living body tissue which is collected in the tissue-sampling section.

In the above-described structure, the outer cylinder member of the tissue-sampling means that is the syringe is detachably coupled to the proximal end portion of the elongated tube body which has the puncture needle at the distal end portion thereof. The shaft-shaped piston member is axially slidably inserted in the cylinder of the outer cylinder member. Further, the living body tissue is collected in the tissue-sampling section, which is disposed at the distal end portion of the tube body, by the suction operation in which the piston member is slid in the outward direction of the outer cylinder member. Subsequently, the living body tissue which is collected in the tissue-sampling section is frozen by the freezing device of the tissue-processing means.

Preferably, the tissue-processing means includes removal means for removing the living body tissue, which is frozen by the freezing device, from the endoscopic therapeutic device.

Preferably, the tissue-processing means includes a biomolecular analysis section which includes a treatment member that subjects the living body tissue, which is collected by the tissue-sampling means, to a process for analysis, and performs biomolecular analysis of the living body tissue by putting the treatment member in contact with the living body tissue.

In the above-described structure, the living body tissue, which is collected by the tissue-sampling means, is subjected to the process for analysis by the treatment member of the tissue-processing means. At this time, the biomolecular analysis section performs biomolecular analysis of the living body tissue by putting the treatment member in contact with the living body tissue.

Preferably, the tissue-processing means includes living body tissue preserving means which is capable of stably preserving biomolecules in the living body tissue in the frozen state, which is frozen by the freezing device.

Preferably, the living body tissue preserving means includes a preservation reagent which is capable of stably preserving the biomolecules in the living body tissue in the frozen state, and means for keeping a low-temperature environment in which decomposition of the biomolecules is prevented by suppressing an activity of a splitting enzyme upon the biomolecules.

Preferably, the tissue-processing means includes a sensor probe which is projectingly provided on the piston member of the syringe, and a biomolecular analysis section which performs biomolecular analysis by making the sensor probe pierce the living body tissue that is collected in the tissue-sampling section.

In the above-described structure, the sensor probe, which is projectingly provided on the piston member of the syringe, is made to pierce the living body tissue that is collected in the tissue-sampling section. Thus, biomolecular analysis is performed by the biomolecular analysis section which is connected to the sensor probe.

According to another aspect of the present invention, a living body tissue analyzing and processing system comprising: tissue-sampling means for sucking and sampling a living body tissue; and tissue-processing means for subjecting the living body tissue, which is collected by the tissue-sampling means, to a process for analysis.

In the above-described structure, the living body tissue is sucked and collected by the tissue-sampling means. Then, the collected living body tissue is subjected to the process for analysis by the tissue-processing means.

Preferably, the tissue-processing means is means for subjecting the living body tissue to at least one of freezing, freezing/drying, division, crushing, ethanol fixation and formalin fixation.

According to another aspect of the present invention, a living body tissue analyzing and processing system comprising: an endoscope which observes an inside of a living body; and an endoscopic therapeutic device which is inserted in the living body through a therapeutic device channel of the endoscope, wherein the endoscopic therapeutic device comprises: an elongated insertion section which is inserted into the living body through the therapeutic device channel; a tubular puncture needle which is disposed at a distal end portion of the insertion section and is made to pierce a living body tissue; tissue-sampling means for sampling the living body tissue in a state in which the puncture needle pierces the living body tissue; and tissue-processing means for subjecting the living body tissue, which is collected by the tissue-sampling means, to a process for analysis.

In the above-described structure, the tubular puncture needle, which is disposed at the distal end portion of the elongated insertion section that is inserted into the living body through the therapeutic device channel of the endoscope which observes the inside of the living body, is made to pierce the living body tissue. The living body tissue is collected by the tissue-sampling means in the state in which the puncture needle pierces the living body tissue. Then, the collected living body tissue is subjected to the process for analysis by the tissue-processing means.

Preferably, the tissue-sampling means comprises a syringe which includes an outer cylinder member which is detachably coupled to a proximal end portion of the tube body, and a shaft-shaped piston member which is axially slidably inserted in a cylinder of the outer cylinder member, the living body tissue being collected into an inside of the outer cylinder member through the puncture needle and the tube body by a suction operation in which the piston member is slid in an outward direction of the outer cylinder member, the tissue-processing means includes a living body tissue freezing mechanism which freezes the living body tissue that is collected in the syringe, and the living body tissue freezing mechanism includes a Peltier element which is mounted in the outer cylinder member of the syringe, and a power supply device which supplies power to the Peltier element.

In the above-described structure, the outer cylinder member of the tissue-sampling means that is the syringe is detachably coupled to the proximal end portion of the elongated tube body which has the puncture needle at the distal end portion thereof. The shaft-shaped piston member is axially slidably inserted in the cylinder of the outer cylinder member. Further, the living body tissue is collected into the inside of the outer cylinder member through the puncture needle and the tube body by the suction operation in which the piston member is slid in the outward direction of the outer cylinder member. Subsequently, power is supplied to the Peltier element from the power supply device. Thereby, the outer cylinder member of the syringe is cooled by the Peltier element, and the living body tissue that is collected in the syringe is frozen.

Preferably, the tissue-sampling means comprises a syringe which includes an outer cylinder member that is detachably coupled to a proximal end portion of the tube body and a shaft-shaped piston member that is axially slidably inserted in a cylinder of the outer cylinder member, the living body tissue being collected into an inside of the outer cylinder member through the puncture needle and the tube body by a suction operation in which the piston member is slid in an outward direction of the outer cylinder member, and the tissue-processing means includes a biomolecular analysis section which includes a treatment member that subjects the living body tissue, which is collected in the syringe, to a process for analysis, and performs biomolecular analysis of the living body tissue by putting the treatment member in contact with the living body tissue.

In the above-described structure, the living body tissue is collected into the inside of the outer cylinder member by the suction operation in which the piston member is slid in the outward direction of the outer cylinder member. Subsequently, biomolecular analysis of the living body tissue is performed by putting the treatment member of the tissue-processing means in contact with the living body tissue that is collected in the outer cylinder member.

According to another aspect of the present invention, a sample-taking method for a tissue analysis process, comprising: a step of inserting an endoscopic therapeutic device, which includes a tubular puncture needle, into a living body through a therapeutic device channel of an endoscope which observes an inside of the living body; a step of making the puncture needle pierce a living body tissue; a tissue-sampling step of sampling the living body tissue into the endoscopic therapeutic device in a state in which the puncture needle pierces the living body tissue; a tissue freezing process step of subjecting the living body tissue, which is collected in the tissue-sampling step, to a freezing process for analysis; and a step of taking out a pellet of a frozen piece of the living body tissue, which is subjected to the freezing process in the tissue freezing process step, from the endoscopic therapeutic device, and transferring the pellet to a tissue analysis process section in a later step.

In the above-described structure, the endoscopic therapeutic device is inserted into the living body through the therapeutic device channel of the endoscope which observes the inside of the living body (therapeutic device insertion step). The puncture needle is made to pierce the living body tissue (puncture needle piercing step). Then, the living body tissue is collected into the endoscopic therapeutic device in the state in which the puncture needle pierces the living body tissue (tissue-sampling step). Subsequently, the living body tissue, which is collected in the tissue-sampling step, is subjected to the freezing process for analysis (tissue freezing process step). Thereafter, the pellet of the frozen piece of the living body tissue, which is subjected to the freezing process in the tissue freezing process step, is taken out from the endoscopic therapeutic device, and the pellet is delivered to the tissue analysis process section in a later step (transfer step).

The present invention can provide an endoscopic therapeutic device, a living body tissue analyzing and processing system, and a sample-taking method for a tissue analysis process, which can make it possible to keep a collected living body tissue in a fresh state and to perform exact diagnosis.

In the present invention, biomolecules are various molecules which constitute a living body, and include relatively high molecular-weight molecules in tissues, for instance, nucleic acids such as DNA and RNA, proteins such as receptors, transcription factors and enzymes, and fats such as hormones, relatively low molecular-weight molecules such as intracellular metabolic products, and foreign-organism-originating biomolecules such as cell-infecting viruses.

In general, in order to stably preserving these biomolecules without losing their functions, it is desirable to instantaneously freeze them. The reason is that decomposition of biomolecules can be prevented by suppressing the activity of splitting enzymes upon various biomolecules included in cells in a low-temperature environment.

The temperatures at this time should preferably be 4°C or below, more preferably -20°C or below, and still more preferably -80°C or below. Since the activity of the DNA splitting enzyme (DNAase) is kept at least at 4°C, the temperatures should desirably be 0°C or below, at which moisture in the tissue is frozen.

It is very important that once freezing is instantaneously effected, the freezing state be maintained until analysis is conducted. If a living body sample is left at high temperatures or freezing/unfreezing is repeated, for example, when the living body sample is transferred, the cells of the living body tissue would be destroyed or the structure/form of cells would be broken. Moreover, at the same time, splitting enzymes of various biomolecules are released and the biomolecules are quickly decomposed. Consequently, the state of the biomolecules in the living body tissue considerably changes from the state at the time when the biomolecules are in the living body, and exact analysis cannot be conducted. It is thus important that the living body tissue, when collected, be instantaneously frozen and transferred to freeze-preservation means or analysis means in the frozen state. Therefore, according to the present invention, the biomolecules in the living body tissue can stably be collected, preserved and analyzed, without deteriorating the condition of the living body tissue at the time of sampling.

### Brief Description of Drawings

FIG. 1A schematically shows the structure of the entirety of an endoscope in which an endoscopic therapeutic device according to a first embodiment of the present invention is assembled;
FIG. 1B is a plan view showing a distal-end structure section of the endoscope according to the first embodiment;
FIG. 2 schematically shows the structure of the endoscopic therapeutic device according to the first embodiment;
FIG. 3 is a block diagram that schematically shows the structure of a control system for a tissue freezing process of the endoscopic therapeutic device according to the first embodiment;
FIG. 4 is an explanatory view for explaining the state in which a pellet of a frozen piece of a living body tissue is taken out of a syringe of the endoscopic therapeutic device according to the first embodiment;
FIG. 5 is a flow chart for explaining a work of sampling a living body tissue by means of the endoscopic therapeutic device according to the first embodiment;
FIG. 6 is a schematic structural view showing a second embodiment of the invention;
FIG. 7A is a schematic structural view showing an endoscopic therapeutic device according to a third embodiment of the invention;
FIG. 7B is a side view showing a metal mesh resin tube in the third embodiment;
FIG. 7C is a perspective view showing a container case of a living body tissue in the third embodiment;
FIG. 8A is a schematic structural view showing the state in which a living body tissue, which is frozen by the endoscopic therapeutic device according to the third embodiment, is contained, together with the container case, in an outer cylinder of a syringe;
FIG. 8B is a perspective view showing the state in which a living body tissue, which is frozen by the endoscopic therapeutic device according to the third embodiment, is taken out of the syringe together with the case;
FIG. 9 is a flow chart for explaining a work of sampling a living body tissue by means of the endoscopic therapeutic device according to the third embodiment;
FIG. 10 schematically shows the structure of the entire endoscope in which an endoscopic therapeutic device according to a fourth embodiment of the present invention is assembled;
FIG. 11A is a longitudinal cross-sectional view of a main part, which shows a frozen state of a living body tissue that is collected in the container case of the endoscopic therapeutic device according to the fourth embodiment;
FIG. 11B is a perspective view showing a container case of a living body tissue in the fourth embodiment;
FIG. 12 is a longitudinal cross-sectional view of a main part, which shows the state in which a sensor needle is pierced in a living body tissue which is frozen in the container case of the endoscopic therapeutic device according to the fourth embodiment;
FIG. 13 is a flow chart for explaining a work of sampling a living body tissue by means of the endoscopic therapeutic device according to the fourth embodiment;
FIG. 14 schematically shows the structure of the entire endoscope in which an endoscopic therapeutic device according to a fifth embodiment of the present invention is assembled;
FIG. 15 is a schematic structural view of a main part of the endoscopic therapeutic device according to the fifth embodiment;
FIG. 16 is a schematic structural view of a main part, which shows the state in which a filter of the endoscopic therapeutic device according to the fifth embodiment is opened;
FIG. 17 is a schematic structural view of the entirety of a living body tissue analyzing and processing system according to a sixth embodiment of the present invention;
FIG. 18 is a schematic structural view of the entirety of a living body tissue analyzing and processing system according to a seventh embodiment of the present invention;
FIG. 19 is a flow chart for explaining a sampling process in a living body tissue analyzing and processing system according to an eighth embodiment of the present invention; and
FIG. 20 is a schematic structural view for explaining a use arraying by the sampling process of the eighth embodiment.

### Best Mode for Carrying Out the Invention

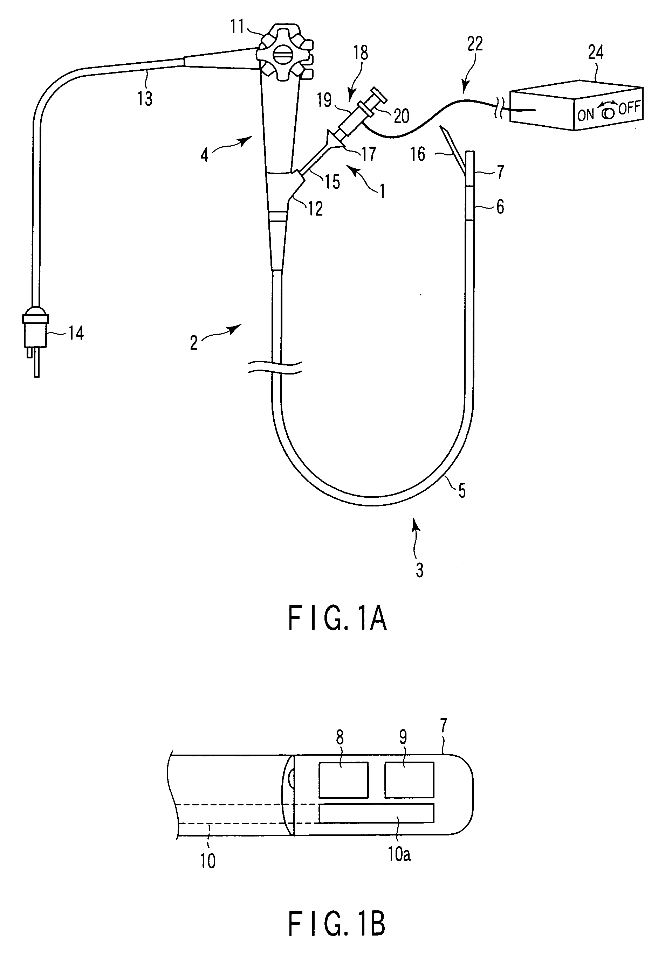

A first embodiment of the present invention will now be described with reference to FIG. 1A to FIG. 5. FIG. 1A schematically shows the structure of the entirety of a flexible side-viewing electronic endoscope 2 in which an endoscopic therapeutic device 1 according to the present embodiment is assembled.

The electronic endoscope 2 includes an elongated insertion section 3. An operation section 4 with a large width is coupled to a proximal end of the insertion section 3. The insertion section 3 is provided with an elongated flexible tube section 5, a bending section 6 that is bendable, and a distal-end structure section 7. A proximal end portion of the flexible tube section 5 is coupled to the operation section 4. A distal end portion of a bending operation wire, which is not shown, is coupled to the bending section 6.

As shown in FIG. 1B, a side surface of the distal-end structure section 7 is provided with an observation window 8 of an observation optical system, an illumination window 9 of an illumination optical system, and a side hole 10a which communicates with a distal end portion of a therapeutic device channel 10. In the observation window 8 of the observation optical system, an objective optical system is provided behind a cover glass. An image pick-up element, such as a CCD, is provided at a focal position of the objective optical system. One end portion of a signal cable is connected to the image pick-up element. In the illumination window 9, a distal end portion of a light guide fiber bundle is provided behind a cover glass. A forceps-raising base is provided in the side hole 10a.

The signal cable of the image pick-up element, the light guide fiber bundle, the therapeutic device channel 10 and the bending operation wire are extended to the operation section 4 side through the inside of the insertion section 3. In addition, the signal cable of the image pick-up element, the light guide fiber bundle, the therapeutic device channel 10 and the bending operation wire are disposed within the insertion section 3 as built-in parts.

The operation section 4 is provided with a bending operation knob 11 and a therapeutic device insertion hole 12. In addition, one end portion of a universal cord 13 is coupled to the operation section 4. The bending operation knob 11 is coupled to a bending operation mechanism which is built in the operation section 4. A proximal end portion of the bending operation wire is coupled to the bending operation mechanism. The bending operation wire is pulled by the operation of the bending operation knob 11 via the bending operation mechanism, and the bending section 6 is remote-operated in the direction of the operation of the bending operation knob 11.

The therapeutic device insertion hole 12 is located at a front side part of the operation section 4. A proximal end portion of the therapeutic device channel 10 is connected to the therapeutic device insertion hole 12. The endoscopic therapeutic device 1 according to the present embodiment is inserted from the therapeutic device insertion hole 12.

A connector section 14 is coupled to the other end portion of the universal cord 13. A proximal end portion of a connection cord for electric signals, which is not shown, is connected to the connector section 14. The other end portion of the connection cord is connected to an electric connector. The connector section 14 is connected to a light source device, and the connector for electric signals is connected to a video processor. The signal cable and the light guide fiber bundle are inserted into the universal cord 13 from the operation section 4. The signal cable is connected to the connector for electric signals. The other end portion of the light guide fiber bundle is connected to the connection section 14. Illumination light, which is emitted from the light source device, is converged on an incidence end face of the light guide fiber bundle, and the illumination light enters the light guide fiber bundle.

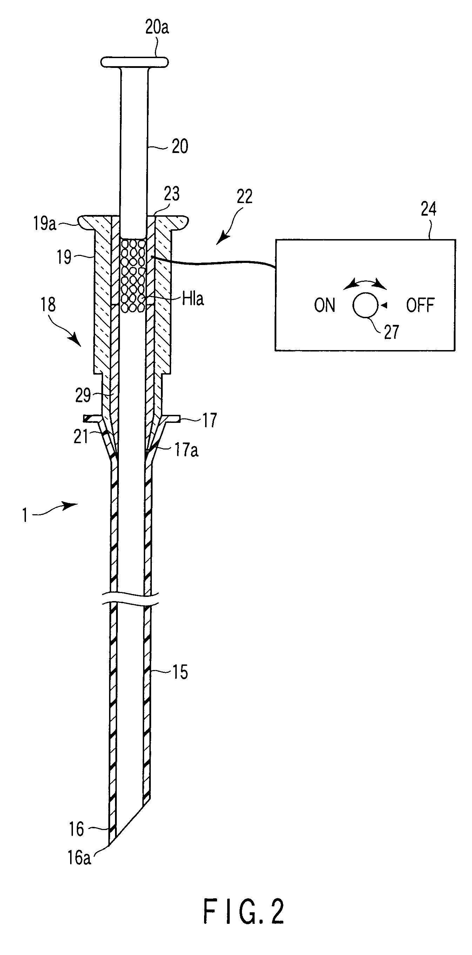

FIG. 2 schematically shows the structure of the endoscopic therapeutic device 1 according to the present embodiment. The endoscopic therapeutic device 1 is provided with an elongated insertion tube (insertion section) 15. The insertion tube 15 is formed of, for example, a resin tube (tube body). The insertion tube 15 is inserted into a living body through the therapeutic device channel 10 of the endoscope 2 which observes the inside of the living body. A tubular puncture needle 16 is disposed at a distal end portion of the insertion tube 15. A needle portion 16a, which is so sharp as to pierce a living body tissue, is formed on the puncture needle 16.

A coupling mouthpiece 17 for an external instrument is formed at a proximal end portion of the insertion tube 15. A taper surface 17a, which has a gradually increasing opening area toward the outside, is formed on the inner peripheral surface of the coupling mouthpiece 17. A syringe (tissue-sampling means) 18 is detachably coupled to the coupling mouthpiece 17 of the insertion tube 15. The syringe 18 includes an outer cylinder member 19 and a shaft-shaped piston member 20. A coupling end portion 21 with a small diameter and a tapered shape is formed at a distal end portion of the outer cylinder member 19. The coupling end portion 21 is inserted and coupled in the coupling mouthpiece 17 of the insertion tube 15. A large-diameter flange portion 19a for finger hooking is formed at a proximal end portion of the outer cylinder member 19. An inside diameter (effective diameter) of the outer cylinder member 19 of the syringe 18 should preferably be set to be as large as possible, so that a large suction pressure can be made to act in the tube body of the elongated insertion tube 15.

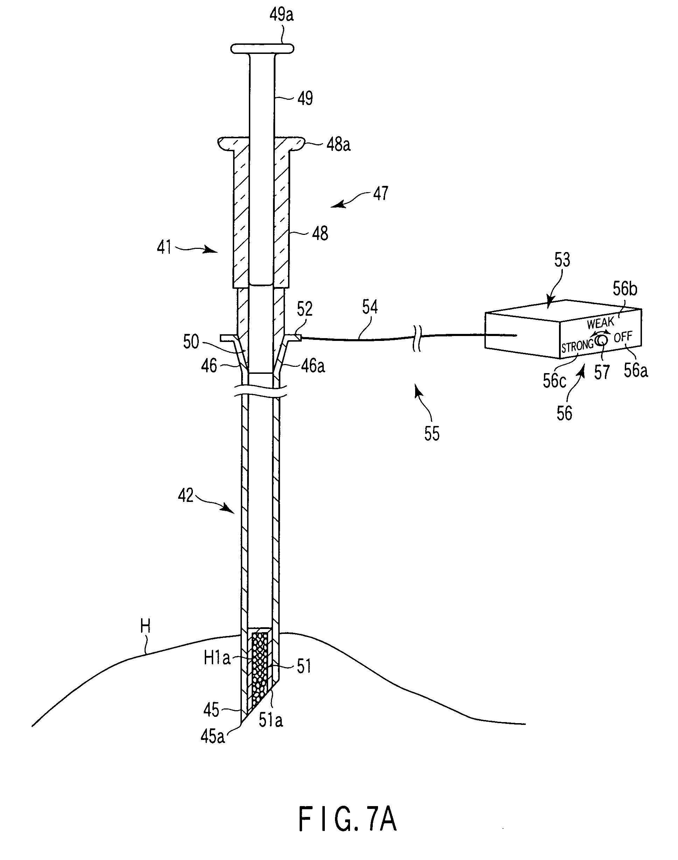

The piston member 20 is axially slidably inserted in the cylinder of the outer cylinder member 19. A large-diameter flange portion 20a for finger hooking, which prevents entrance into the cylinder of the outer cylinder member 19, is formed at an outer end portion of the piston member 20. In the state in which the puncture needle 16 of the endoscopic therapeutic device 1 pierces a living body tissue H (see FIG. 7A), the living body tissue H is collected and taken into the inside of the outer cylinder member 19 through the conduit of the puncture needle 16 and insertion tube 15 by the suction operation in which the piston member 20 is slid in an outward direction (upward direction in FIG. 2) of the outer cylinder member 19 from the position where the piston member 20 is inserted in the outer cylinder member 19.

Besides, the endoscopic therapeutic device 1 of the present embodiment is provided with a freezing device (tissue processing means) 22 which subjects the living body tissue H collected by the syringe 18 to a process for analysis, for example, living body tissue freezing in this embodiment, by which the living body tissue H is frozen. The freezing device 22 includes a Peltier element 23 which is mounted in the outer cylinder member 19, and a power supply device 24 which supplies power to the Peltier element 23.

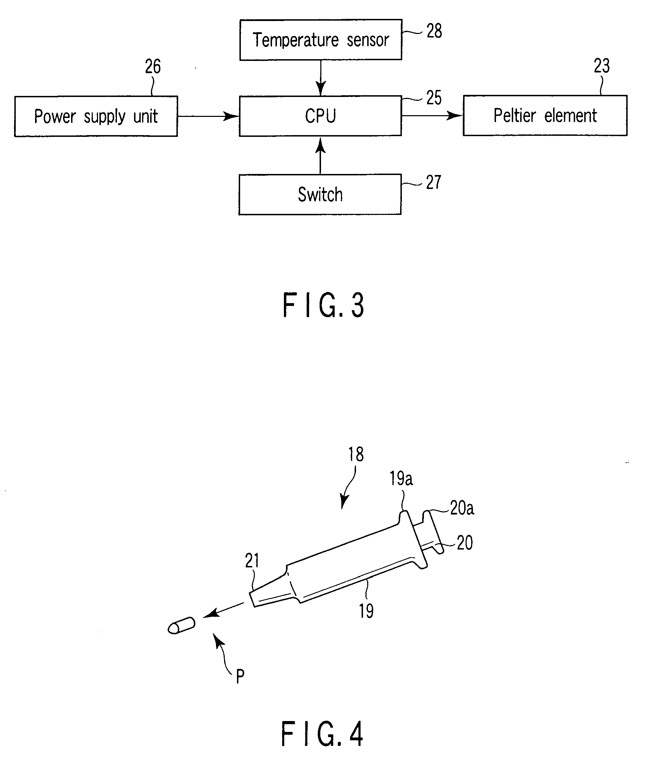

FIG. 3 is a block diagram that schematically shows the structure of a control system for a tissue freezing process, which is assembled in the power supply device 24. A CPU 25, which constitutes, for example, control means of a computer, is built in the power supply device 24. A power supply unit 26, a switch 27, a temperature sensor 28, Peltier element 23 and a memory device (not shown) are connected to the CPU 25. The switch 27 executes the on/off operation of the driving state of the Peltier element 23. The temperature sensor 28 measures the temperature of the living body tissue H that is collected by the syringe 18.

In the meantime, in the syringe 18, a heat conduction pipe 29, which is formed of, e.g. a metallic material with high heat conductivity, is mounted on the inner peripheral surface of the outer cylinder member 19. The heat conduction pipe 29 is attached in a state of contact with the Peltier element 23. The temperature sensor 28 is attached to the heat conduction pipe 29.

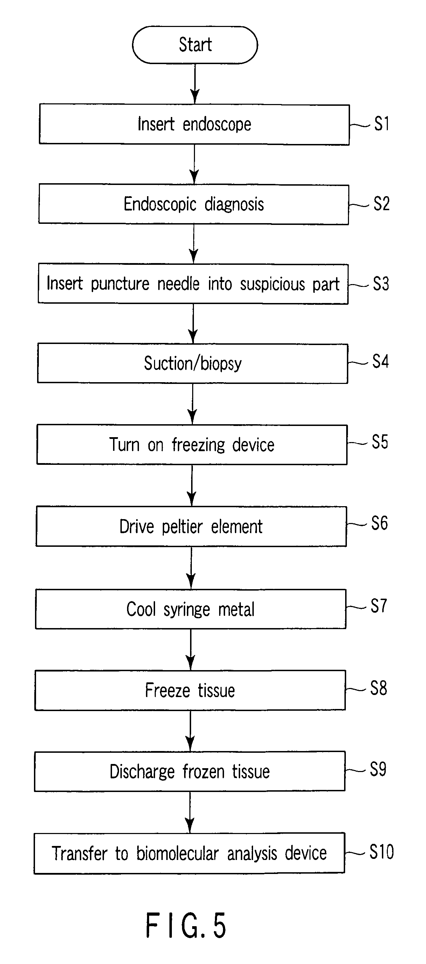

Next, the operation of the above-described structure is described. The operation at the time of use of the endoscopic therapeutic device 1 according to the present embodiment is described with reference to a flow chart of FIG. 5. To begin with, the electronic endoscope 2 is inserted into the body of a patient (step S1). At this time, the insertion section 3 of the electronic endoscope 2 is inserted into the body from the distal-end structure section 7. During the work of inserting the endoscope 2, illumination light is radiated from the illumination window 9 of the endoscope 2, and the inside of the body cavity is observed through the observation widow 8. An endoscopic image, which is observed through the observation window 8, is displayed on a monitor (not shown).

In this state, the insertion section 3 of the endoscope 2 is inserted up to a target part within the body cavity. Thereafter, diagnosis of the target part within the body cavity is conducted by the electronic endoscope 2 (step S2). At this time, an inspection target part, which is, for example, a suspicious diseased part, within the body cavity is determined. This is followed by an operation of inserting the endoscopic therapeutic device 1 into the body cavity through the therapeutic device channel 10 of the endoscope 2.

At the time of inserting the endoscopic therapeutic device 1, the insertion tube 15 of the endoscopic therapeutic device 1 is inserted into the therapeutic device channel 10 from the therapeutic device insertion hole 12 of the endoscope 2. The insertion tube 15 of the endoscopic therapeutic device 1 is inserted into the living body through the therapeutic device channel 10.

The insertion tube 15 of the endoscopic therapeutic device 1, which is guided to the distal end side through the therapeutic device channel 10, is projected into the body from the side hole 10a. At this time, the direction of projection of the insertion tube 15 of the endoscopic therapeutic device 1 is adjusted by the forceps-raising base, and the direction of projection of the insertion tube 15 is remote-controlled so as to agree with a desired direction.

Thereafter, the puncture needle 16 of the endoscopic therapeutic device 1 is made to pierce the inspection target part which is a suspicious diseased part and is determined by the endoscopic diagnosis in step S2 (step S3). At this time, a sample of a living body tissue H of the inspection target part is taken into the puncture needle 16. In this state, the syringe 18 is operated. At this time, the piston member 20 is slid in an outward direction (upward direction in FIG. 2) of the outer cylinder member 19 from the position where the piston member 20 is inserted in the outer cylinder member 19. At the time of this operation, a suction force for sucking the living body tissue H acts through the conduit of the puncture needle 16 and insertion tube 15. Thus, by this suction operation, as shown in FIG. 2, the living body tissue H1a of the inspection target part is collected into the outer cylinder member 19 of the syringe 18 (step S4). The living body tissue H1a, which is collected at this time, includes blood and viscous membrane.

Subsequently, the switch 27 of the power supply device 24 is turned on to drive the freezing device 22 (step S5). When the freezing device 22 is driven, power is supplied to the Peltier element 23 and the Peltier element 23 is driven (step S6). Thereby, the external cylinder member 19 of the syringe 18 is cooled by the Peltier element 23 (step S7), and the living body tissue that is collected in the syringe 18 is frozen (step S8). At this time, the freezing temperature of the living body tissue is set at, e.g. about -10°C to -60°C. For example, in the case where dry ice is used as a freezing substance of the freezing device 22, the freezing temperature of the living body tissue can be set at -80°C, and in the case where liquid nitrogen is used, the freezing temperature of the living body tissue can be set at about -196°C. Besides, the living body tissue that is collected in the syringe 18 may be treated with a toriton 100(trademark) surface active agent as a cell membrane lysis solution, so that the analysis of the living body tissue may become easier.

After the living body tissue that is collected in the syringe 18 is frozen, the syringe 18 is removed from the coupling mouthpiece 17 of the insertion tube 15. In this state, as shown in FIG. 4, the piston member 20 of the syringe 18 is pushed and a pellet P of the living body tissue that is frozen in the outer cylinder member 19 is taken out (step S9). Thereafter, the pellet P of the frozen living body tissue is delivered to a tissue analysis process unit in a later step, such as a biomolecular analysis device (step S10). Thus, the sample-taking work for the tissue analysis process is completed.

The following advantageous effect can be obtained by the above-described structure. Specifically, in the endoscopic therapeutic device 1 according to the present embodiment, the insertion tube 15 is inserted into the living body through the therapeutic device channel 10 of the endoscope 2, and the puncture needle 16 at the distal end portion of the insertion tube 15 is made to pierce the living body tissue. In this state, the living body tissue is sucked and collected by the syringe 18, and then the collected living body tissue is frozen by the freezing device 22 and is processed for analysis. Thus, the collected living body tissue can be kept in a fresh state. Therefore, since the living body tissue can be delivered in the fresh state to the tissue analysis process unit in a later step, such as a biomolecular analysis device, it becomes possible to perform, with high precision, various inspections of the living body tissue, such as cytological diagnosis, histological diagnosis and biomolecular analysis.

In the structure of the present embodiment, the endoscopic therapeutic device 1 is inserted into the body through the therapeutic device channel 10 of the flexible side-viewing electronic endoscope 2. The invention, however, is not limited to this structure. For example, in an alternative structure, the endoscopic therapeutic device 1 may be inserted into the body through a therapeutic device channel of a flexible forward-viewing electronic endoscope 2.

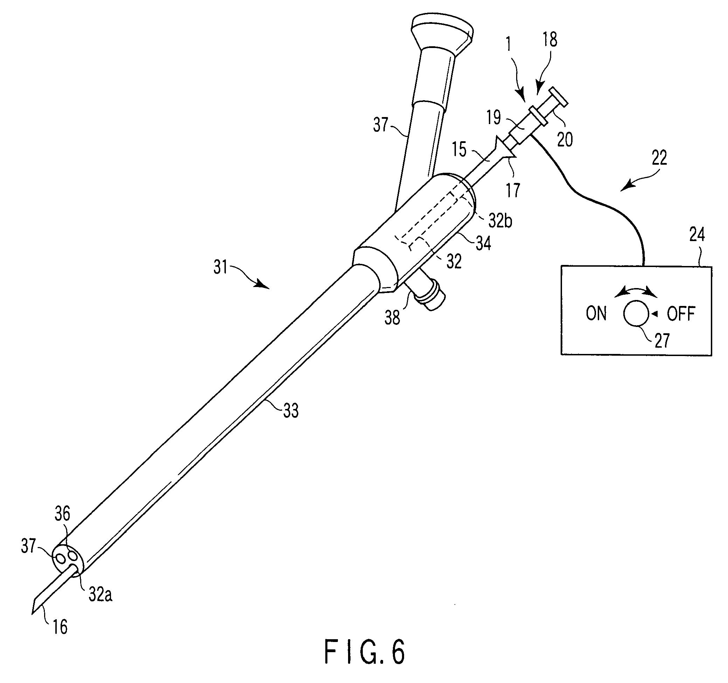

FIG. 6 shows a second embodiment of the present invention. In the structure of this embodiment, the endoscopic therapeutic device 1 shown in the first embodiment (see FIG. 1 to FIG. 5) is inserted into the body through a therapeutic device channel 32 of a rigid endoscope 31. Since the structure of the endoscopic therapeutic device 1 is the same as in the first embodiment, the parts common to those in the first embodiment are denoted by like reference numerals, and a description thereof is omitted.

The rigid endoscope 31 of this embodiment is provided with an elongated straight rigid insertion section 33 which is formed of, for example, a metallic tube. A large-diameter operation section 34 is provided at a proximal end portion of the insertion section 33. An observation window 35 of an observation optical system, an illumination window 36 of an illumination optical system and a distal-end opening portion 32a of the therapeutic device channel 32 are formed in a distal end portion of the insertion section 33.

An eyepiece section 37 for observation and a light guide connection mouthpiece 38 are projectingly provided on an outer peripheral surface of the operation section 34. An image transmission optical system, which transmits an observation image that is incident from the observation window 35 to the eyepiece section 37, is provided between the eyepiece section 37 and the observation window 35. Further, a light guide which transmits illumination light is provided between the light guide connection mouthpiece 38 and the illumination window 36.

A rear-end opening portion 32b of the therapeutic device channel 32 is formed at a terminal end portion of the operation section 34. The insertion tube 15 of the endoscopic therapeutic device 1 is inserted into the therapeutic device channel 32 from the rear-end opening portion 32b, and inserted into the living body through the therapeutic device channel 32.

Next, the operation of the present embodiment with the above-described structure is described. When the endoscopic therapeutic device 1 of this embodiment is used, the rigid endoscope 31 is inserted in advance into the body of a patient. When the rigid endoscope 31 is inserted, a trocar, for instance, is used. The trocar includes an outer sheath and a puncture needle which is detachably inserted in this outer sheath. In the state in which the puncture needle is inserted and assembled in the outer sheath, the trocar is made to pierce, for example, the abdominal wall of the patient. Subsequently, in the state in which the puncture needle is drawn out of the outer sheath, only the outer sheath is let to stay in the state in which the outer sheath pierces the abdominal wall. The rigid endoscope 31 is inserted into the body through this outer sheath.

After the rigid endoscope 31 is inserted, the insertion tube 15 of the endoscopic therapeutic device 1 is inserted into the living body through the therapeutic device channel 32 of the rigid endoscope 31. Subsequently, by the same operation as in the first embodiment, the living body tissue H is collected by the endoscopic therapeutic device 1. After the living body tissue that is collected in the syringe 18 is frozen, the same work as in the first embodiment is performed, and the piston member 20 of the syringe 18 is pushed and a pellet P of the living body tissue that is frozen in the outer cylinder member 19 is taken out of the syringe 18.

In the present embodiment, too, the insertion tube 15 of the endoscopic therapeutic device 1 is inserted into the living body through the therapeutic device channel 32 of the rigid endoscope 31, and the puncture needle 16 at the distal end portion of the insertion tube 15 is made to pierce the living body tissue. In this state, the living body tissue is sucked and collected by the syringe 18, and then the collected living body tissue can be frozen by the freezing device 22 and processed for analysis. Thus, like the first embodiment, the collected living body tissue can be kept in a fresh state. Therefore, since the living body tissue can be delivered in the fresh state to the tissue analysis process unit in a later step, such as a biomolecular analysis device, it becomes possible to perform, with high precision, various inspections of the living body tissue, such as cytological diagnosis, histological diagnosis and biomolecular analysis.

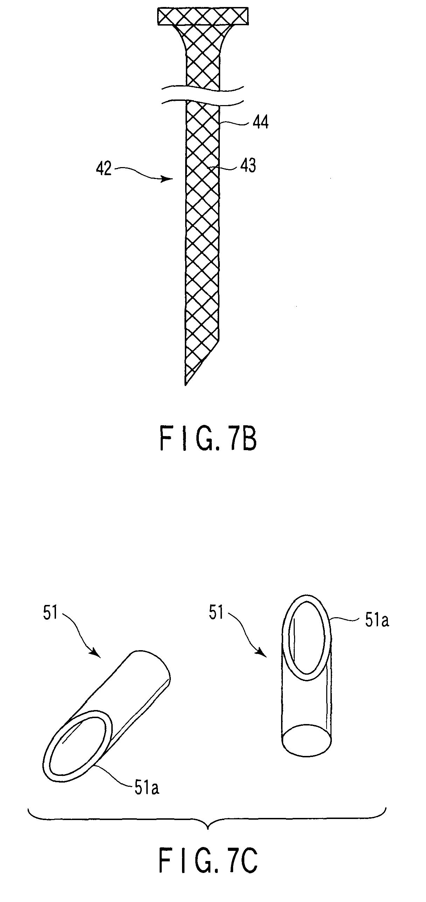

FIG. 7A to FIG. 9 show a third embodiment of the present invention. FIG. 7A schematically shows the structure of an endoscopic therapeutic device 41 according to this embodiment. This endoscopic therapeutic device 41 is provided with an elongated insertion tube (insertion section) 42. As shown in FIG. 7B, the insertion tube 42 is formed of, for example, a metallic mesh resin tube in which a metallic mesh 44, which is formed by weaving metal wires in a lattice fashion, is mounted on an outer surface of a resin tube (tube body) 43. The resin tube 43 is formed of a material with high heat conductivity. The metallic mesh 44 may be integrally formed in the state in which the metallic mesh 44 is buried in the resin tube 43. A silicone coat, for example, is formed on the outer surface of the insertion tube 42, and thereby the metallic mesh 44 is prevented from being exposed to the outer surface of the insertion tube 42.

The insertion tube 42 is inserted into a living body through a therapeutic device channel of an endoscope which observes the inside of the living body, for example, the therapeutic device channel 10 of the flexible side-viewing electronic endoscope 2 of the first embodiment (see FIG. 1A to FIG. 5) or the therapeutic device channel 32 of the rigid endoscope 31 of the second embodiment (see FIG. 6). A tubular puncture needle 45 is disposed at a distal end portion of the insertion tube 42. A needle portion 45a, which is so sharp as to pierce a living body tissue, is formed on the puncture needle 45.

A coupling mouthpiece 46 for an external instrument is formed at a proximal end portion of the insertion tube 42. A taper surface 46a, which has a gradually increasing opening area toward the outside, is formed on the inner peripheral surface of the coupling mouthpiece 46. A syringe 47 is detachably coupled to the coupling mouthpiece 46 of the insertion tube 42. The syringe 47 includes an outer cylinder member 48 and a shaft-shaped piston member 49. A coupling end portion 50 with a small diameter and a tapered shape is formed at a distal end portion of the outer cylinder member 48. The coupling end portion 50 is inserted and coupled in the coupling mouthpiece 46 of the insertion tube 42. A large-diameter flange portion 48a for finger hooking is formed at a proximal end portion of the outer cylinder member 48. An inside diameter (effective diameter) of the outer cylinder member 48 of the syringe 47 should preferably be set to be as large as possible, so that a large suction pressure can be made to act in the tube body of the elongated insertion tube 42.

The piston member 49 is axially slidably inserted in the cylinder of the outer cylinder member 48. A large-diameter flange portion 49a for finger hooking, which prevents entrance into the cylinder of the outer cylinder member 48, is formed at an outer end portion of the piston member 49.

In the endoscopic therapeutic device 41 of the present embodiment, a living body tissue container case 51, which is formed of a resin with high heat conductivity, is inserted and set in the distal end portion of the insertion tube 42. As shown in FIG. 7C, the living body tissue container case 51 is a bottomed cylindrical case having a cylindrical body which is closed at one end. The container case 51 should preferably be sliceable. On the open end side of the container case 51, an opening end portion 51a with an inclined surface, which is obliquely cut in accordance with the shape of the puncture needle 45 at the distal end of the insertion tube 42, is formed. In the state in which the container case 51 is set in the insertion tube 42, the bottom side of the container case 51 is inserted into the insertion tube 42 (i.e. the bottom side is located on the upper side in FIG. 7A), and the opening end portion 51a is positioned in the state in which the opening end portion 51a is overlapped with a distal-end opening portion of the puncture needle 45.

A Peltier element 52 is mounted on the coupling mouthpiece 46 of the insertion tube 42. A connection cord 54 for connection to a power supply device 53, which supplies power, is connected to the Peltier element 52. Driving power is supplied to the Peltier element 52 from the power supply device 53 via the connection cord 54. When the Peltier element 52 is driven, the metallic mesh 44 of the insertion tube 42 is cooled, and also the container case 51 is cooled by heat conduction. Thereby, the living body tissue H1, which is collected in the container case 51, is frozen. The Peltier element 52 and the power supply device 53 constitute a freezing device (tissue processing means) 55 which subjects the living body tissue H collected in the container case 51 to a process for analysis, for example, living body tissue freezing in this embodiment, by which the living body tissue H is frozen.

The power supply device 53 is provided with a switch 56 which executes the on/off operation of the driving state of the Peltier element 52. The switch 56 includes an operation dial 57 which performs a selective change-over operation between, for example, an off (stop) position 56a, a weak position 56b and a strong position 56c. When the operation dial 57 is set at the off (stop) position 56a, selective change-over switching is effected to an unfreezing state. When the operation dial 57 is set at the weak position 56b, selective change-over switching is effected to a weak freezing state in which the living body tissue H1 is frozen at temperatures of, e.g. about -10°C to -30°C. When the operation dial 57 is set at the strong position 56c, selective change-over switching is effected to a strong freezing state in which the living body tissue H1 is frozen at temperatures of, e.g. about -60°C to -80°C.

The power supply device 53 is provided with a control system (see FIG. 3) for a tissue freezing process, which has the same structure as in the first embodiment. In accordance with the operation of the operation dial 57 of the switch 56, the driving state of the Peltier element 52 can be selectively changed over, as described above, between the three states, i.e. the unfreezing state, weak freezing state and strong freezing state.

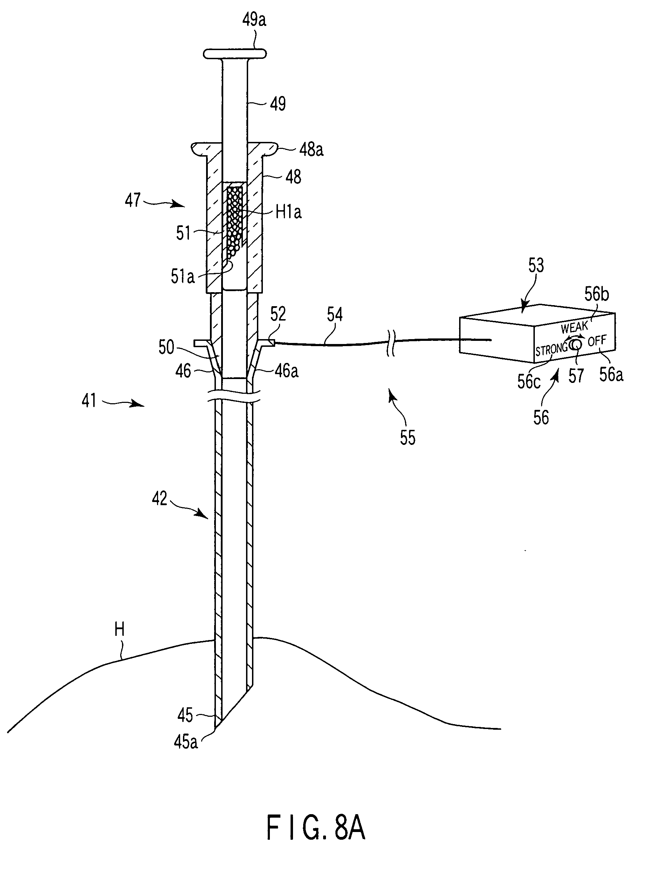

In the endoscopic therapeutic device 41 of the present embodiment, the syringe 47 is used in retrieving the living body tissue H that is collected in the container case 51. Specifically, as shown in FIG. 7A, when the puncture needle 45 at the distal end of the insertion tube 42 is made to pierce the body inner wall (living body tissue) H of the organ, such as the stomach, the living body tissue H1 is collected in the container case 51. At this time, the piston member 49 of the syringe 47 is held in the push-in position in which the piston member 49 is pushed in the outer cylinder member 48. After the living body tissue H1 is frozen, as shown in FIG. 8A, the frozen living body tissue H, together with the container case 51, is sucked and retrieved into the outer cylinder member 48 of the syringe 47 through the conduit of the puncture needle 45 and insertion tube 42 by the suction operation in which the piston member 49 of the syringe 47 is slid in an outward direction (upward direction in FIG. 7) of the outer cylinder member 48 from the position where the piston member 49 is inserted in the outer cylinder member 48.

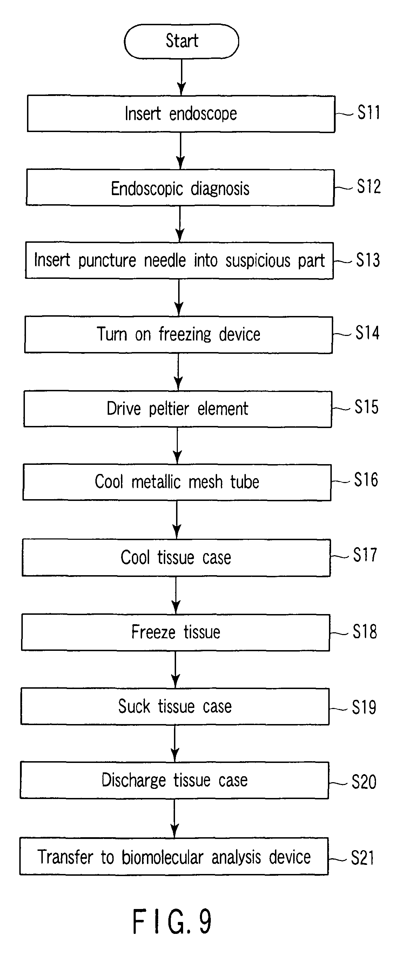

Next, the operation of the above-described structure is described. The operation at the time of use of the endoscopic therapeutic device 41 according to the present embodiment is described with reference to a flow chart of FIG. 9. To begin with, like the first embodiment, the electronic endoscope 2 is inserted into the body of a patient (step S11). During the work of inserting the endoscope 2, illumination light is radiated from the illumination window 9 of the endoscope 2, and the inside of the body cavity is observed through the observation widow 8. An endoscopic image, which is observed through the observation window 8, is displayed on a monitor (not shown).

In this state, the insertion section 3 of the endoscope 2 is inserted up to a target part within the body cavity. Thereafter, diagnosis of the target part within the body cavity is conducted by the electronic endoscope 2 (step S12). At this time, an inspection target part, which is, for example, a suspicious diseased part, within the body cavity is determined. This is followed by an operation of inserting the endoscopic therapeutic device 41 into the living body through the therapeutic device channel 10 of the endoscope 2.

At the time of inserting the endoscopic therapeutic device 41, the insertion tube 42 of the endoscopic therapeutic device 41 is inserted into the therapeutic device channel 10 from the therapeutic device insertion hole 12 of the endoscope 2. The insertion tube 42 of the endoscopic therapeutic device 41 is inserted into the living body through the therapeutic device channel 10.

The insertion tube 42 of the endoscopic therapeutic device 41, which is guided to the distal end side through the therapeutic device channel 10, is projected into the body from the side hole 10a. At this time, the direction of projection of the insertion tube 42 of the endoscopic therapeutic device 41 is adjusted by the forceps-raising base, and the direction of projection of the insertion tube 42 is remote-controlled so as to agree with a desired direction.

Thereafter, the puncture needle 45 of the endoscopic therapeutic device 41 is made to pierce the inspection target part which is a suspicious diseased part and is determined by the endoscopic diagnosis in step S12 (step S13). When the puncture needle 45 of the endoscopic therapeutic device 41 pierces the body inner wall (living body tissue) H of the organ, such as the stomach, the living body tissue H is collected in the container case 51.

Subsequently, in this state, the operation dial 57 of the switch 56 of the power supply device 53 is operated. At this time, the operation dial 57 is changed over to one of the weak position 56b and strong position 56c, and the power supply device 53 is driven (step S14).

When the power supply device 53 is driven, power is supplied to the Peltier element 52 in accordance with the operation of the operation dial 57, and the Peltier element 52 is driven (step S15). When the Peltier element 52 is driven, the metallic mesh 44 of the insertion tube 42 is cooled (step S16). Further, the container case 51 is cooled by heat conduction (step S17). Thereby, the living body tissue H1 that is collected in the container case 51 is frozen (step S18). At this time, in the case where the operation dial 57 of the power supply device 53 is set at the weak position 56b, the living body tissue H1 is frozen in the weak freezing state at freezing temperatures of, e.g. about -10°C to -30°C. In the case where the operation dial 57 is set at the strong position 56c, the living body tissue H1 is frozen in the strong freezing state at freezing temperatures of, e.g. about -60°C to -80°C. In this case, too, the living body tissue that is collected in the container case 51 may be treated with a toriton 100(trademark) surface active agent as a cell membrane lysis solution, so that the analysis of the living body tissue may become easier.

Further, after the living body tissue collected in the container case 51 is frozen, the syringe 47 is operated. At this time, as shown in FIG. 7A, the piston member 49 of the syringe 47 is held in the push-in position in which the piston member 49 is pushed in the outer cylinder member 48. After the living body tissue H is frozen, as shown in FIG. 8A, the suction operation is performed to slide the piston member 49 of the syringe 47 from the inside of the outer cylinder member 48 in the outward direction of the outer cylinder member 48. By this suction operation, the frozen living body tissue H1, together with the container case 51, is sucked and retrieved into the outer cylinder member 48 of the syringe 47 through the conduit of the puncture needle 45 and insertion tube 42 (step S19).

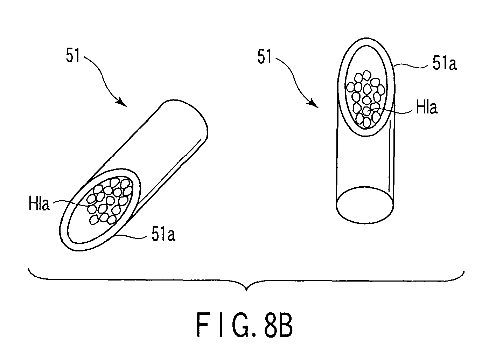

Thereafter, the syringe 47 is removed from the coupling mouthpiece 46 of the insertion tube 42. In this state, the piston member 49 of the syringe 47 is pushed and a pellet P of the frozen living body tissue H1 is taken out of the syringe 47 together with the container case 51. Subsequently, as shown in FIG. 8B, the pellet P of the frozen living body tissue contained in the container case 51 is discharged and taken out of the container case 51 (step S20). Following this, the pellet P of the frozen living body tissue is delivered to a tissue analysis process unit in a later step, such as a biomolecular analysis device (step S21). Thus, the sample-taking work for the tissue analysis process is completed.

The following advantageous effect can be obtained by the above-described structure. Specifically, in the endoscopic therapeutic device 41 according to the present embodiment, the insertion tube 42 is inserted into the living body through the therapeutic device channel 10 of the endoscope 2, and the puncture needle 45 at the distal end portion of the insertion tube 42 is made to pierce the living body tissue. Thereby, the living body tissue is collected in the container case 51. Then, the collected living body tissue H1 is frozen by the freezing device 55 and is processed for analysis. Thus, the collected living body tissue H1 can be kept in a fresh state. Therefore, since the living body tissue H1 can be transferred in the fresh state to the tissue analysis process unit in a later step, such as a biomolecular analysis device, it becomes possible to perform, with high precision, various inspections of the living body tissue H1, such as cytological diagnosis, histological diagnosis and biomolecular analysis.

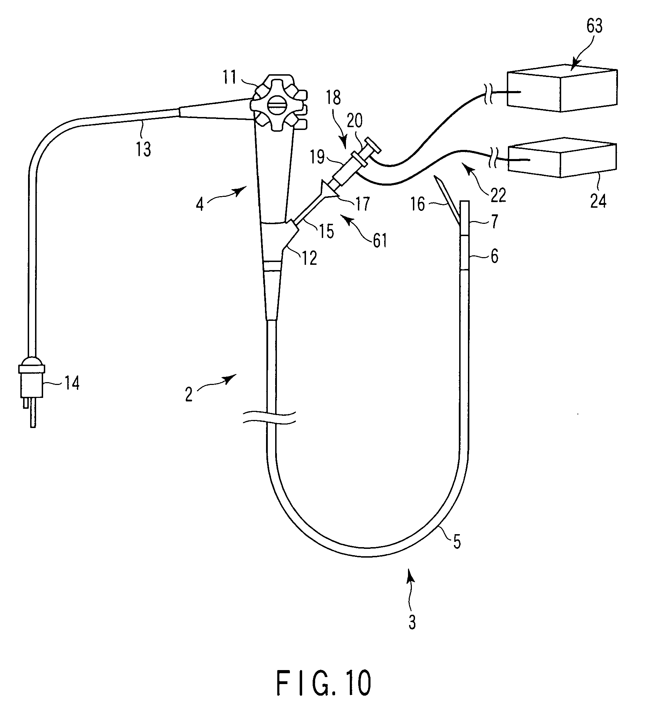

FIG. 10 to FIG. 13 show a fourth embodiment of the present invention. An endoscopic therapeutic device 61 of the present embodiment is configured such that a biomolecular analysis device 62 is added to the endoscopic therapeutic device 1 of the first embodiment (see FIG. 1A to FIG. 5). In the other respects, the structure of the endoscopic therapeutic device 61 of the present embodiment is the same as the structure of the endoscopic therapeutic device 1 of the first embodiment. Thus, the parts common to those of the endoscopic therapeutic device 1 of the first embodiment are denoted by like reference numerals, and a description thereof is omitted.

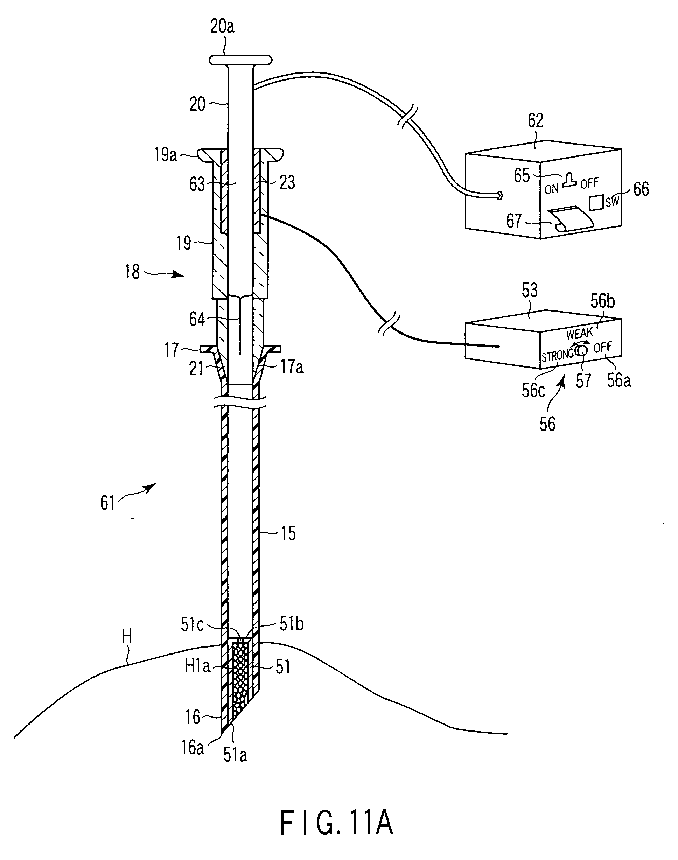

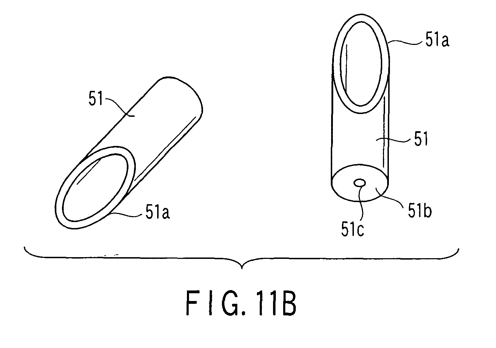

FIG. 10 is a schematic structural view showing the state in which the endoscopic therapeutic device 61 of the present embodiment is inserted in the therapeutic device channel 10 of the electronic endoscope 2. FIG. 11 schematically shows the entire structure of the endoscopic therapeutic device 61 of the present embodiment.

In the endoscopic therapeutic device 61 of the present embodiment, a biomolecular analysis sensor 63 is built in the piston member 20 of the syringe 18. A proximal end portion of a sensor probe 64, which projects forward from a distal end portion (inner end portion) of the piston member 20, is coupled to the biomolecular analysis sensor 63. The sensor probe 64 is disposed at the axial center position of the distal end face of the piston member 20.

A container case 51, which is substantially similar to the living body tissue container case 51 of the third embodiment (see FIG. 7A to FIG. 9), is inserted and set in the tubular puncture needle 16 at the distal end portion of the insertion tube 15. As shown in FIG. 11B, a through-hole 51c is formed at a central position of a bottom portion 51b of the container case 51. The sensor probe 64 can be inserted in the through-hole 51c of the container case 51.

An external biomolecular analysis device 62 is connected to the biomolecular analysis sensor 63. The biomolecular analysis device 62 includes a power switch 65, a data print switch 66 and a data printer 67.

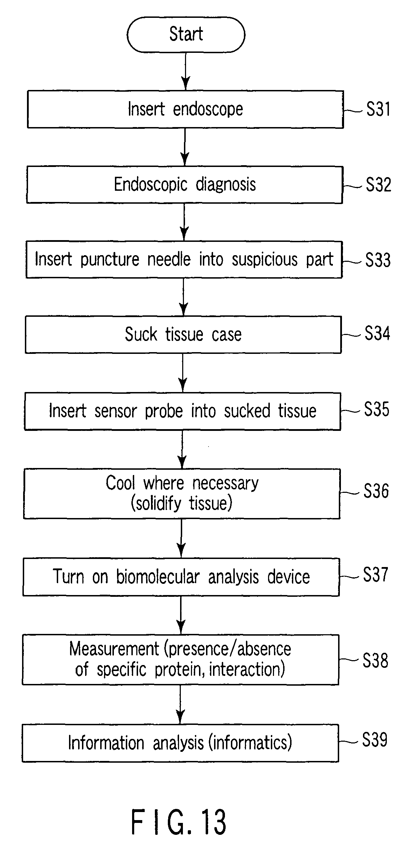

Next, the operation of the above-described structure is described. The operation at the time of use of the endoscopic therapeutic device 61 according to the present embodiment is described with reference to a flow chart of FIG. 13. To begin with, like the first embodiment, the electronic endoscope 2 is inserted into the body of a patient (step S31). During the work of inserting the endoscope 2, illumination light is radiated from the illumination window 9 of the endoscope 2, and the inside of the body cavity is observed through the observation widow 8. An endoscopic image, which is observed through the observation window 8, is displayed on a monitor (not shown).

In this state, the insertion section 3 of the endoscope 2 is inserted up to a target part within the body cavity. Thereafter, diagnosis of the target part within the body cavity is conducted by the electronic endoscope 2 (step S32). At this time, an inspection target part, which is, for example, a suspicious diseased part, within the body cavity is determined. This is followed by an operation of inserting the endoscopic therapeutic device 61 into the living body through the therapeutic device channel 10 of the endoscope 2.

At the time of inserting the endoscopic therapeutic device 61, the insertion tube 15 of the endoscopic therapeutic device 61 is inserted into the therapeutic device channel 10 from the therapeutic device insertion hole 12 of the endoscope 2. The insertion tube 15 of the endoscopic therapeutic device 61 is inserted into the living body through the therapeutic device channel 10.

The insertion tube 15 of the endoscopic therapeutic device 61, which is guided to the distal end side through the therapeutic device channel 10, is projected into the body from the side hole 10a. At this time, the direction of projection of the insertion tube 15 of the endoscopic therapeutic device 61 is adjusted by the forceps-raising base, and the direction of projection of the insertion tube 15 is remote-controlled so as to agree with a desired direction.

Thereafter, the puncture needle 16 of the endoscopic therapeutic device 61 is made to pierce the inspection target part which is a suspicious diseased part and is determined by the endoscopic diagnosis in step S32 (step S33). When the puncture needle 16 of the endoscopic therapeutic device 61 pierces the body inner wall (living body tissue) H of the organ, such as the stomach, the living body tissue H1 is collected in the container case 51. At this time, the piston member 20 of the syringe 18 is held in the push-in position in which the piston member 20 is pushed in the outer cylinder member 19, as shown in FIG. 11A.

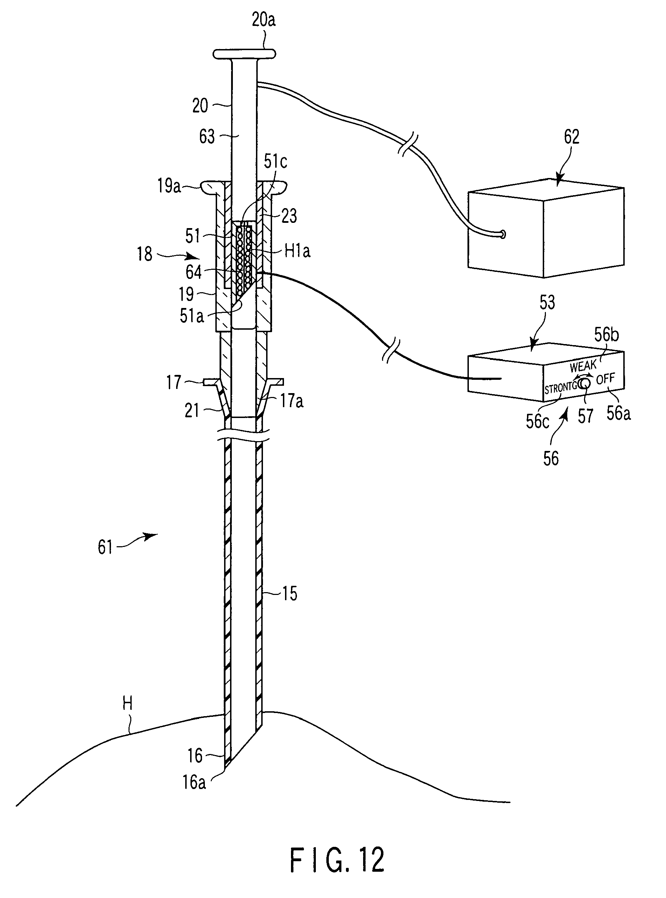

Subsequently, in this state, as shown in FIG. 12, the suction operation is performed to slide the piston member 20 of the syringe 18 from the inside of the outer cylinder member 19 in the outward direction of the outer cylinder member 19. By this suction operation, the living body tissue H1, together with the container case 51, is sucked into the outer cylinder member 19 of the syringe 18 through the conduit of the insertion tube 15 (step S34). At this time, the sensor probe 64 is inserted into the through-hole 51c of the container case 51, and the sensor probe 64 is made to pierce the living body tissue H1 within the container case 51 (step S35).

Subsequently, where necessary, the operation dial 57 of the switch 56 of the power supply device 53 is operated. At this time, the operation dial 57 is changed over to one of the weak position 56b and strong position 56c, and the power supply device 53 is driven.

When the power supply device 53 is driven, power is supplied to the Peltier element 23 in accordance with the operation of the operation dial 57, and the Peltier element 23 is driven. When the Peltier element 23 is driven, the living body tissue H1 that is collected in the container case 51 is frozen (step S36).

Thereafter, the power switch 65 of the biomolecular analysis device 62 is turned on (step S37). At this time, by the sensor probe 64 of the biomolecular analysis sensor 63, biomolecular analysis, for example, the presence/absence of specific protein or the measurement of interactions, is conducted on the pellet P of the frozen living body tissue H1 that is contained in the container case 51 (step S38).

Following the measurement in step S38, the data printer 67 of the biomolecular analysis device 62 is driven and the information analysis of the biomolecular analysis device 62 is performed (step S39). In the information analysis, for example, analysis is conducted on information such as the probability of metastasis (prediagnosis after a surgical operation), optimization of therapeutic measures, and sensitivity to anticancer drugs. Thus, the tissue analysis process work is completed.

The following advantageous effect can be obtained by the above-described structure. Specifically, in the endoscopic therapeutic device 61 according to the present embodiment, the insertion tube 15 is inserted into the living body through the therapeutic device channel 10 of the endoscope 2, and the puncture needle 16 at the distal end portion of the insertion tube 15 is made to pierce the living body tissue. Thereby, the living body tissue is collected in the container case 51. Then, the sensor probe 64 of the biomolecular analysis sensor 63 is made to pierce the collected living body tissue H1, thus being able to conduct the biomolecular analysis on the pellet P of the living body tissue H1 contained in the container case 51, for example, the presence/absence of specific protein or the measurement of interactions. Therefore, since the living body tissue H1 can quickly be subjected to the biomolecular analysis in a later step in the fresh state, it becomes possible to perform, with high precision, various inspections of the living body tissue H1, such as cytological diagnosis, histological diagnosis and biomolecular analysis.

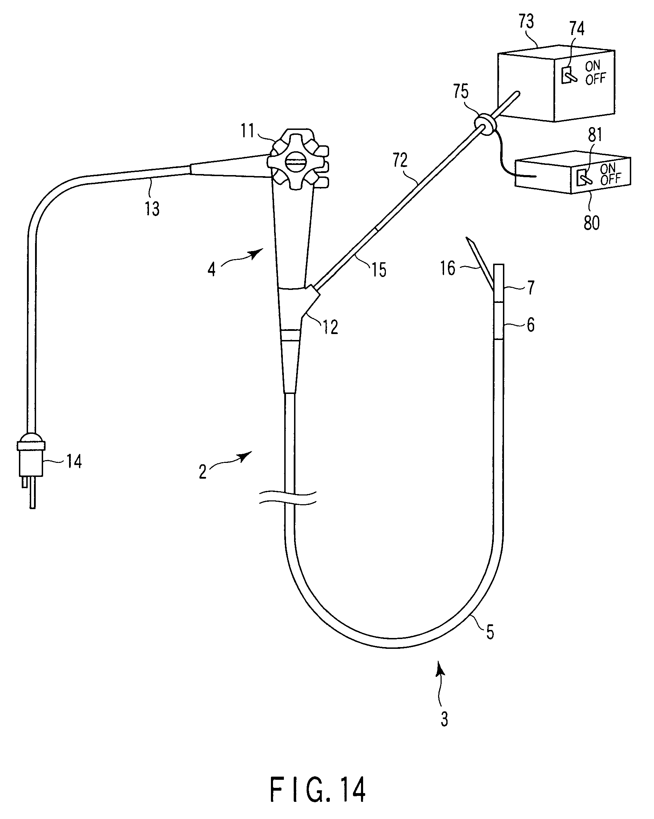

FIG. 14 to FIG. 16 show a fifth embodiment of the present invention. The present embodiment is provided with an endoscopic therapeutic device 71 having a structure that is different from the structure of the endoscopic therapeutic device 1 of the first embodiment (see FIG. 1A to FIG. 5).

In the endoscopic therapeutic device 71 of the present embodiment, the syringe 18 in the first embodiment is replaced with a suction device 73 as suction means for use in sampling a living body tissue. In the other respects, the structure of the endoscopic therapeutic device 71 of the present embodiment is the same as the structure of the endoscopic therapeutic device 1 of the first embodiment. Thus, the parts common to those of the endoscopic therapeutic device 1 of the first embodiment are denoted by like reference numerals, and a description thereof is omitted.

Specifically, in the endoscopic therapeutic device 71, a distal end portion of a suction conduit 72 for suction is coupled to a proximal end portion of the insertion tube 15. The suction device 73 is coupled to a proximal end portion of the suction conduit 72. The suction device 73 is provided with a switch 74 for an on/off operation. A suction probe is composed of the insertion tube 15 and the suction conduit 72.

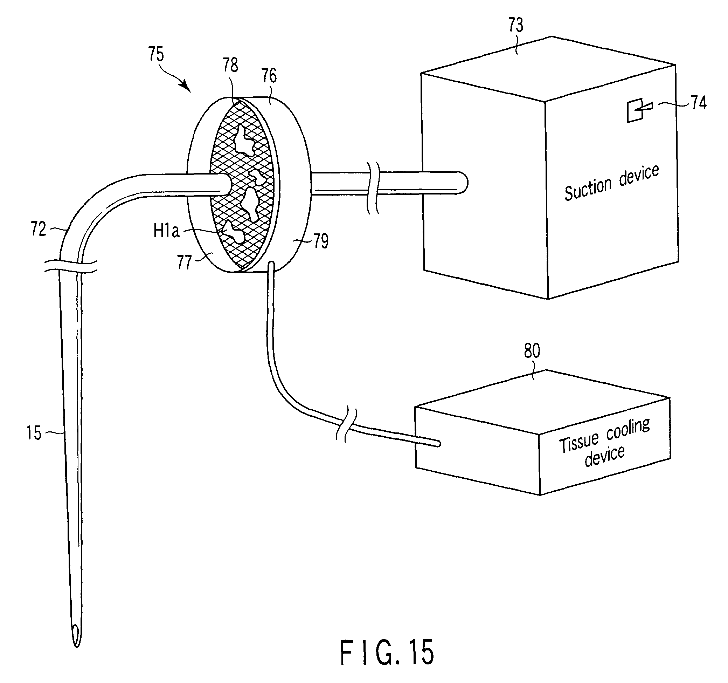

A filter 75 for taking a sample is provided near a connection part between the suction conduit 72 and the suction device 73. As shown in FIG. 15, the filter 75 is provided with a filter container 76 having a bottomed cylinder shape. A transparent cap 77 is detachably attached to an opening face of the filter container 76.

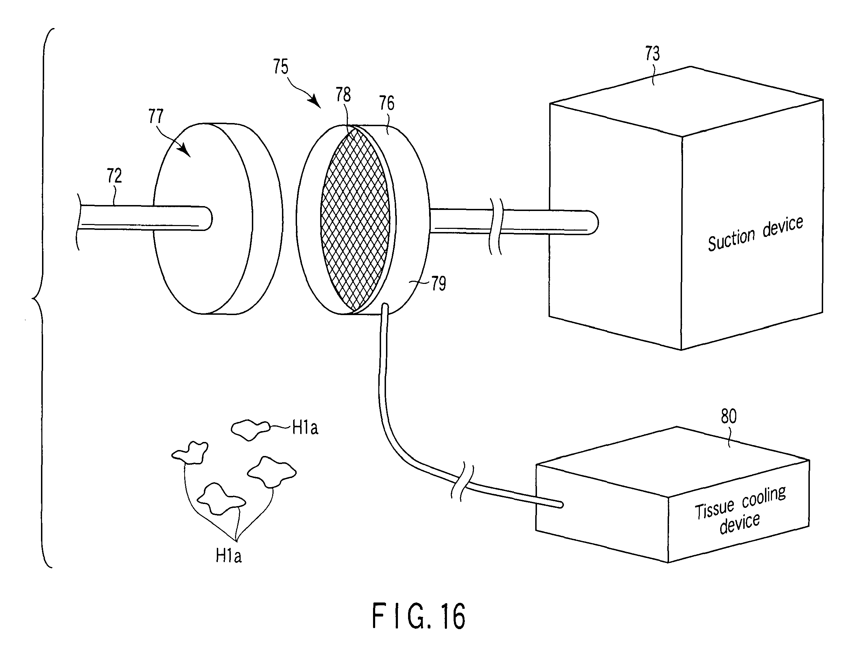

Further, as shown in FIG. 16, a net-like partition plate 78, which constitutes a filter body, is provided within the filter container 76. The inside of the filter container 76 is partitioned by the partition plate 78 into a suction chamber on the suction device 73 side (bottom part side) and a sample-taking chamber on the insertion tube 15 side (opening face side). A living body tissue H1 of a sample, which is sucked in the filter container 76, is trapped by the partition plate 78 and taken in the sample-taking chamber.

A peripheral wall portion of the filter container 76 is formed of a Peltier-element-containing ring 79. The Peltier-element-containing ring 79 is connected to a tissue cooling device 80 in which a power supply device for supplying power to the Peltier element is built in. The tissue cooling device 80 is provided with a switch 81 for an on/off operation. When the switch 81 is turned on, power is supplied to the Peltier element of the Peltier-element-containing ring 79, thus driving the Peltier element. The living body tissue H1 of the sample taken in the filter container 76 is frozen by the Peltier-element-containing ring 79.

Next, the operation of the above-described structure is described. In the endoscopic therapeutic device 71 of the present embodiment, an operation of inserting the insertion tube 15 of the endoscopic therapeutic device 71 into the living body through the therapeutic device channel 10 of the endoscope 2 is performed according to the same procedure as with the endoscopic therapeutic device 1 of the first embodiment.

Subsequently, the puncture needle 16 of the endoscopic therapeutic device 71 is made to pierce an inspection target part which is a suspicious diseased part and is determined by endoscopic diagnosis. At this time, a sample of the living body tissue H1 of the inspection target part is taken in the conduit of the puncture needle 16. In this state, the switch 74 of the suction device 73 is turned on, and a suction force acts in the insertion tube 15 and suction conduit 72. Thereby, the sample of the living body tissue H1 of the inspection target part, which has been taken in the conduit of the puncture needle 16, is sucked into the filter container 76 through the insertion tube 15 and suction conduit 72.

Further, the living body tissue H1 of the sample that is sucked in the filter container 76 is trapped by the partition plate 78, and taken in the sample-taking chamber. In this state, the switch 81 of the tissue cooling device 80 is turned on. Thereby, power is supplied to the Peltier element of the Peltier-element-containing ring 79, thus driving the Peltier element. The living body tissue H1 of the sample taken in the filter container 76 is frozen by the Peltier-element-containing ring 79. In an alternative configuration, the tissue cooling device 80 may be driven at the same time as the driving of the suction device 73, so that the living body tissue H1 of the sample that is sucked in the filter container 76 may immediately be frozen when the living body tissue H1 is trapped by the partition plate 78.

After the living body tissue H1 that is taken in the filter container 76 is frozen, the transparent cap 77 is removed from the filter container 76. In this state, the frozen living body tissue H1 is taken out of the filter container 76. Then, the frozen living body tissue H1 is delivered to a tissue analysis process unit in a later step, such as a biomolecular analysis device. Thus, the sample-taking work for the tissue analysis process is completed.

The following advantageous effect can be obtained by the above-described structure. Specifically, in the endoscopic therapeutic device 71 according to the present embodiment, the insertion tube 15 is inserted into the living body through the therapeutic device channel 10 of the endoscope 2, and the puncture needle 16 at the distal end portion of the insertion tube 15 is made to pierce the living body tissue. Then, a suction force is caused to act in the insertion tube 15 and suction conduit 72 by the suction device 73, and the living body tissue H1 is collected in the filter container 76. Thereafter, the collected living body tissue H1 is frozen by the Peltier-element-containing ring 79 and processed for analysis. Thus, the collected living body tissue can be kept in a fresh state. Therefore, since the living body tissue can be transferred in the fresh state to the tissue analysis process unit in a later step, such as a biomolecular analysis device, it becomes possible to perform, with high precision, various inspections of the living body tissue, such as cytological diagnosis, histological diagnosis and biomolecular analysis.

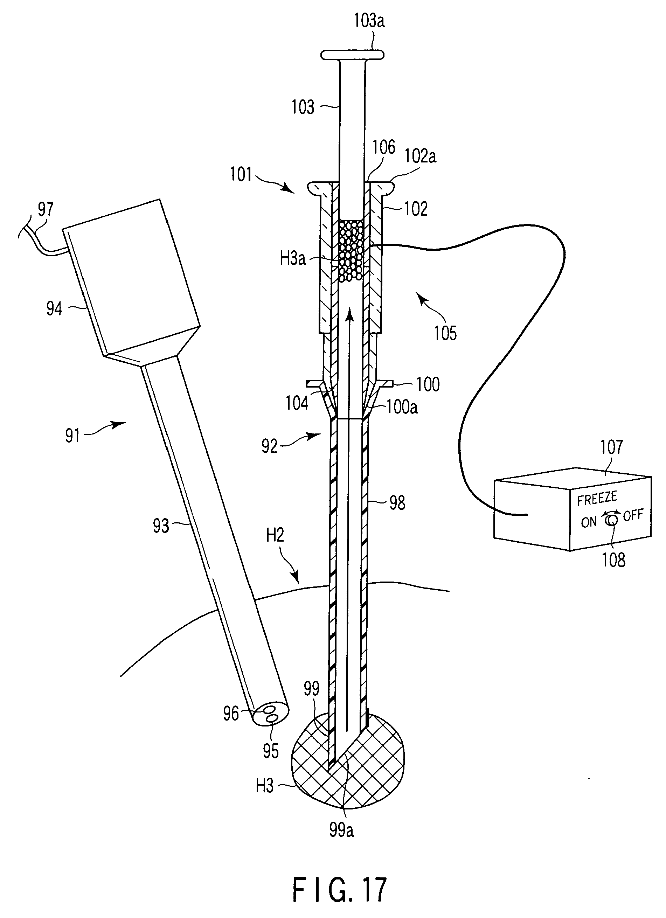

FIG. 17 shows a sixth embodiment of the present invention. In the present embodiment, the invention is applied to a laparoscopic surgical operation system. In FIG. 17, reference numeral 91 denotes a rigid endoscope which is a laparoscope, and numeral 92 denotes a therapeutic device for sampling a living body tissue.

The rigid endoscope 91 includes a straight, elongated rigid insertion section 93 which is formed of, for example, a metallic tube. An operation section 94 with a large diameter is provided at a proximal end of the insertion section 93. A distal end portion of the insertion section 93 is provided with an observation window 95 of an observation optical system and an illumination window 96 of an illumination optical system. An objective lens and an image pick-up element, such as a CCD, which is disposed at a focal position of the objective lens, are provided behind the observation window 95. A light guide for transmitting illumination light is provided behind the illumination window 96.

One end portion of a universal cord 97 is coupled to an outer peripheral surface of the operation section 94. A connector section, which is connected to a light source device and a video processor (not shown), is coupled to the other end portion of the universal cord 97.

The therapeutic device 92 for living body tissue sampling is provided with an elongated tube (insertion section) 98. The insertion tube 98 is inserted into the living body through an outer sheath of a trocar (not shown) which is made to pierce, in advance, a living body tissue H2 of an abdominal wall part of a patient. A tubular puncture needle 99 is disposed at a distal end portion of the insertion tube 98. A needle portion 99a, which is so sharp as to pierce a target living body tissue H3 such as a tumor, is formed on the puncture needle 99.

A coupling mouthpiece 100 for an external instrument is formed at a proximal end portion of the insertion tube 98. A taper surface 100a, which has a gradually increasing opening area toward the outside, is formed on the inner peripheral surface of the coupling mouthpiece 100. A syringe (tissue-sampling means) 101 is detachably coupled to the coupling mouthpiece 100 of the insertion tube 98. The syringe 101 includes an outer cylinder member 102 and a shaft-shaped piston member 103. A coupling end portion 104 with a small diameter and a tapered shape is formed at a distal end portion of the outer cylinder member 102. The coupling end portion 104 is inserted and coupled in the coupling mouthpiece 100 of the insertion tube 98. A large-diameter flange portion 102a for finger hooking is formed at a proximal end portion of the outer cylinder member 102. An inside diameter (effective diameter) of the outer cylinder member 102 of the syringe 101 should preferably be set to be as large as possible, so that a large suction pressure can be made to act in the conduit of the elongated insertion tube 98.

The piston member 103 is axially slidably inserted in the cylinder of the outer cylinder member 102. A large-diameter flange portion 103a for finger hooking, which prevents entrance into the cylinder of the outer cylinder member 102, is formed at an outer end portion of the piston member 103. In the state in which the puncture needle 99 of the insertion tube 98 pierces the living body tissue H3 in the body of the patient, a sample H3a of the living body tissue H3, such as a tumor, is collected and taken into the inside of the outer cylinder member 102 through the conduit of the puncture needle 99 and insertion tube 98 by the suction operation in which the piston member 103 is slid in an outward direction (upward direction in FIG. 17) of the outer cylinder member 102 from the position where the piston member 103 is inserted in the outer cylinder member 102.

Besides, the therapeutic device 92 for living body tissue sampling, according to the present embodiment, is provided with a freezing device (tissue processing means) 105 which subjects the sample H3a of the living body tissue H3 that is collected by the syringe 101 to a process for analysis, which is a living body tissue freezing process in the present embodiment, by which the sample H3a of the living body tissue H3 is frozen. Like the first embodiment, the freezing device 105 includes a Peltier element 106 which is mounted in the outer cylinder member 102, and a power supply device 107 which supplies power to the Peltier element 106.

Next, the operation of the above-described structure is described. In the present embodiment, the insertion section 93 of the rigid endoscope 91 is inserted into the body through the outer sheath of the trocar (not shown) which is made to pierce, in advance, the living body tissue H2 of the abdominal wall part of the patient. Similarly, the insertion tube 98 of the therapeutic device 92 for living body tissue sampling is inserted into the living body through the outer sheath of the trocar (not shown) which is made to pierce, in advance, the living body tissue H2 of the abdominal wall part of the patient. In the observation visual field of the rigid endoscope 91, the work of making the puncture needle 99 at the distal end portion of the insertion tube 98 pierce the target living body tissue H3 is performed. At this time, the sample H3a of the living body tissue H3 of the inspection target part is taken into the conduit of the puncture needle 99. In this state, the syringe 101 is operated. At this time, the piston member 103 is slid in an outward direction (upward direction in FIG. 17) of the outer cylinder member 102 from the position where the piston member 103 is inserted in the outer cylinder member 102. At the time of this operation, a suction force for sucking the sample H3a of the living body tissue H3 acts through the conduit of the puncture needle 99 and insertion tube 98. Thus, by this suction operation, as shown in FIG. 17, the sample H3a of the living body tissue H3 of the inspection target part is collected into the outer cylinder member 102 of the syringe 101.

Subsequently, the switch 108 of the power supply device 107 is turned on to drive the freezing device 105. When the freezing device 105 is driven, power is supplied to the Peltier element 106 and the Peltier element 106 is driven. Thereby, the external cylinder member 102 of the syringe 101 is cooled by the Peltier element 106, and the sample H3a of the living body tissue H3 that is collected in the syringe 101 is frozen.

After the sample H3a of the living body tissue H3 that is collected in the syringe 101 is frozen, the syringe 101 is removed from the coupling mouthpiece 100 of the insertion tube 98. In this state, the piston member 103 of the syringe 101 is pushed and the pellet P of the sample H3a of the living body tissue H3, which is frozen in the outer cylinder member 102, is taken out. Thereafter, the pellet P of the frozen sample H3a of the living body tissue H3 is transferred to a tissue analysis process unit in a later step, such as a biomolecular analysis device. Thus, the sample-taking work for the tissue analysis process is completed.

The following advantageous effect can be obtained by the above-described structure. Specifically, in the laparoscopic surgical operation system of the present embodiment, in the observation visual field of the rigid endoscope 91, the puncture needle 99 at the distal end portion of the insertion tube 98 of the therapeutic device 92 for living body tissue sampling is made to pierce the target living body tissue H3, and in this state the sample H3a of the living body tissue H3 is sucked and retrieved by the syringe 101. Then, the collected living body tissue is frozen by the freezing device 105 and processed for analysis. Thus, the retrieved sample H3a of the living body tissue H3 can be kept in a fresh state. Therefore, since the sample H3a of the living body tissue H3 can be transferred in the fresh state to the tissue analysis process unit in a later step, such as a biomolecular analysis device, it becomes possible to perform, with high precision, various inspections of the sample H3a of the living body tissue H3, such as cytological diagnosis, histological diagnosis and biomolecular analysis.

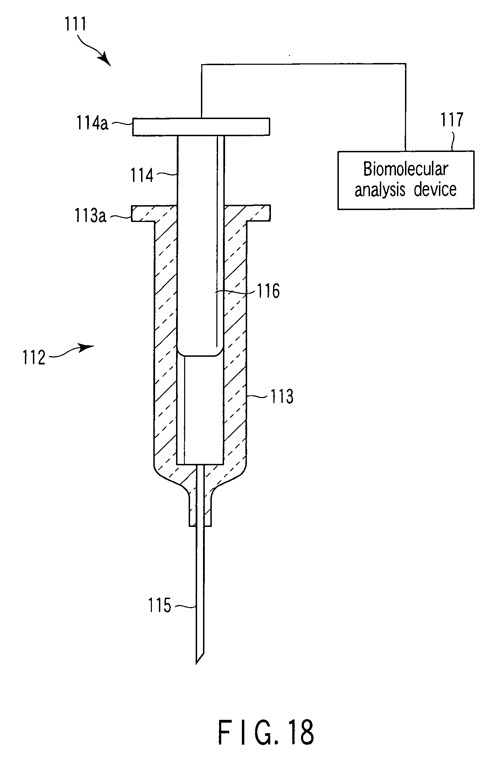

FIG. 18 shows a seventh embodiment of the present invention. In the present embodiment, a living body tissue analyzing and processing system 111, which is different from that of each of the preceding embodiments, is provided. In the living body tissue analyzing and processing system 111 of this embodiment, a syringe 112 is mainly used. The syringe 112 includes an outer cylinder member 113 and a shaft-shaped piston member 114. A tapered small-diameter coupling end portion 113a is formed at a distal end portion of the outer cylinder member 113. A proximal end portion of a thin tubular injection needle 115 is fixed to the coupling end portion 113a. A large-diameter flange portion 113a for finger hooking is formed at a proximal end portion of the outer cylinder member 113.

The piston member 114 is axially slidably inserted in the cylinder of the outer cylinder member 113. A large-diameter flange portion 114a for finger hooking, which prevents entrance into the cylinder of the outer cylinder member 113, is formed at an outer end portion of the piston member 114. When the syringe 112 is used, a distal end portion of the injection needle 115 is made to pierce the living body tissue H (see FIG. 7). In this state, the living body tissue H is collected and taken into the inside of the outer cylinder member 113 through the conduit of the injection needle 115 by the suction operation in which the piston member 114 is slid in an outward direction (upward direction in FIG. 18) of the outer cylinder member 113 from the position where the piston member 114 is inserted in the outer cylinder member 113.

A biomolecular analysis device 117, which performs biomolecular analysis of the living body tissue H that is collected by the syringe 112, is connected to the living body tissue analyzing and processing system 111 of the present embodiment. A detector 116, which performs, for example, light detection, fluorescence detection and polarization detection, is built in the syringe 112. Such a configuration may be adopted that a chemical solution, such as a cell lysis solution or a DNA extraction solution, is added to the living body tissue H, which is collected in the outer cylinder member 113 of the syringe 112, and the living body tissue is subjected to pretreatment.

Next, the operation of the above-described structure is described. When the living body tissue analyzing and processing system 111 of the present embodiment is used, the distal end portion of the injection needle 115 of the syringe 112 is made to pierce the living body tissue H. The living body tissue H is collected and taken into the inside of the outer cylinder member 113 through the conduit of the injection needle 115 by the suction operation in which the piston member 114 is slid in an outward direction (upward direction in FIG. 18) of the outer cylinder member 113 from the position where the piston member 114 is inserted in the outer cylinder member 113.

Subsequently, light detection, fluorescence detection and polarization detection, for example, are performed by the detector 116 in the syringe 112, and the detection data is transmitted to the biomolecular analysis device 117. Based on the transmitted detection data, various biomolecular analysis processes are carried out by the biomolecular analysis device 117.

The following advantageous effect can be obtained by the above-described structure. Specifically, in the living body tissue analyzing and processing system 111 of the present embodiment, after the living body tissue H is sucked and collected by the syringe 112, the light detection, fluorescence detection and polarization detection, for example, of the collected living body tissue are performed by the detector 116 in the syringe 112. Based on the detection data, the biomolecular analysis device 117 performs the biomolecular analysis process. Thus, the collected living body tissue H in the fresh state can be subjected to the biomolecular analysis. Therefore, it is possible to perform various kinds of biomolecular analysis of the living body tissue H with high precision.

In the present invention, the living body tissue H is collected from inside the body. This embodiment, however, is also applicable to the case of sampling cells of the skin, blood from outside the body.

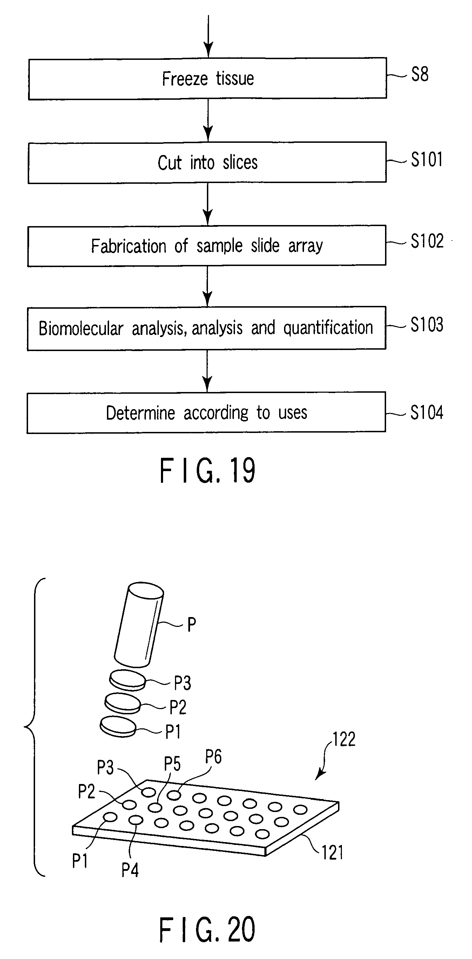

FIG. 19 and FIG. 20 show an eighth embodiment of the present invention. The present embodiment shows a modification of the sample process after the sample of the living body tissue H1 is taken by the endoscopic therapeutic device 1 of the first embodiment (see FIG. 1A to FIG. 5).

FIG. 19 is a flow chart showing a use in which the frozen pellet P of the sample of the living body tissue H1, which is taken by using the endoscopic therapeutic device 1 of the first embodiment, is arrayed. In this case, step S101 follows the step S8 in the flow chart of FIG. 5 of the first embodiment. In step S101, as shown in FIG. 20, a slicing work is performed to cut the frozen pellet P into round slice pieces p1, p2, p3, ....

The step S101 is followed by a subsequent step S102. In step S102, the slice pieces p1, p2, p3, ..., which are cut in step S101, are orderly arranged on a base board 121 such as a glass plate. Thereby, a sample slide array 122 is fabricated.

Then, the next step S103 is executed. In step S103, the sample slide array 122 is subjected to genome proteome analysis, analysis, quantification, etc. Thereby, analysis with positional information is conducted. Further, in the next step S104, determination according to uses is performed.

The following advantageous effect can be obtained by the above-described structure. Specifically, in the present embodiment, while the frozen pellet P is being continuously pushed out of the syringe 112, a minute slice cutting means, such as a diamond blade, is successively driven. Thereby, the frozen pellet P is cut into slice pieces p1, p2, p3, ..., each having a thickness of about 10 nm to 50 µm, under a microscope. The slice pieces p1, p2, p3, ..., are orderly arranged on the base board 121 such as a glass plate. Thereby, the sample slide array 122, which formed by arraying, is fabricated. At this time, in order to facilitate fixation of slice pieces, the base board 121 may be subjected, in advance, to surface treatment or coated with a binder reagent. Until the sample slide array 122 is used, the sample slide array 122 is kept in a frozen or dry state. At the time of use, the sample slide array 122 is unfrozen or wetted with a solution, and then treatments, such as genome proteome analysis, analysis, quantification, etc., are performed. Thereby, analysis with positional information is conducted, and determination according to uses is performed. Techniques for applying frozen tissues to micro-arrays, which are other than those described above, are disclosed in, e.g. PCT National Publication No. 2004-500891 and Jpn. Pat. Appln. KOKAI Publication No. 2-161334.

Needless to say, the present invention is not limited to the above-described embodiments, and various modifications may be made without departing from the spirit of the invention.

### Industrial Applicability

The present invention is effective in technical fields in which an endoscopic therapeutic device and a living body tissue analyzing and processing system are used, and in technical fields in which a sample-taking method for a tissue analysis process is carried out to perform an inspection such as a biomolecular analysis process of a living body tissue.

## Claims

1. An endoscopic therapeutic device comprising:
an elongated insertion section which is inserted into a living body through a therapeutic device channel of an endoscope which observes an inside of the living body;
a tubular puncture needle which is disposed at a distal end portion of the insertion section and is made to pierce a living body tissue;
tissue-sampling means for sampling the living body tissue in a state in which the puncture needle pierces the living body tissue; and
tissue-processing means for subjecting the living body tissue, which is collected by the tissue-sampling means, to a process for analysis.

2. The endoscopic therapeutic device according to claim 1, wherein the insertion section includes an elongated tube body, and the puncture needle is formed at a distal end portion of the tube body,
the tissue-sampling means comprises a syringe which includes an outer cylinder member which is detachably coupled to a proximal end portion of the tube body, and a shaft-shaped piston member which is axially slidably inserted in a cylinder of the outer cylinder member, the living body tissue being collected into an inside of the outer cylinder member through the puncture needle and the tube body by a suction operation in which the piston member is slid in an outward direction of the outer cylinder member, and
the tissue-processing means includes a freezing device which freezes the living body tissue which is collected in the syringe.

3. The endoscopic therapeutic device according to claim 2, wherein the freezing device includes a Peltier element which is mounted in the outer cylinder member of the syringe, and a power supply device which supplies power to the Peltier element.

4. The endoscopic therapeutic device according to claim 1, wherein the insertion section includes an electrically conductive elongated tube body, and the puncture needle is formed at a distal end portion of the tube body,
the tissue-sampling means comprises a syringe, which includes an outer cylinder member that is detachably coupled to a proximal end portion of the tube body and a shaft-shaped piston member that is axially slidably inserted in a cylinder of the outer cylinder member, and a tissue-sampling section which is disposed at the distal end portion of the tube body, and the tissue-sampling means includes a tissue-sampling mechanism which samples the living body tissue into an inside of the tissue-sampling section through the puncture needle by a suction operation in which the piston member is slid in an outward direction of the outer cylinder member, and
the tissue-processing means includes a freezing device which freezes the living body tissue which is collected in the tissue-sampling section.

5. The endoscopic therapeutic device according to claim 2 or 4, wherein the tissue-processing means includes removal means for removing the living body tissue, which is frozen by the freezing device, from the endoscopic therapeutic device.

6. The endoscopic therapeutic device according to claim 1, wherein the tissue-processing means includes a biomolecular analysis section which includes a treatment member that subjects the living body tissue, which is collected by the tissue-sampling means, to a process for analysis, and performs biomolecular analysis of the living body tissue by putting the treatment member in contact with the living body tissue.

7. The endoscopic therapeutic device according to claim 2 or 4, wherein the tissue-processing means includes living body tissue preserving means which is capable of stably preserving biomolecules in the living body tissue in the frozen state, which is frozen by the freezing device.

8. The endoscopic therapeutic device according to claim 7, wherein the living body tissue preserving means includes a preservation reagent which is capable of stably preserving the biomolecules in the living body tissue in the frozen state, and means for keeping a low-temperature environment in which decomposition of the biomolecules is prevented by suppressing an activity of a splitting enzyme upon the biomolecules.

9. The endoscopic therapeutic device according to claim 2, wherein the tissue-processing means includes a sensor probe which is projectingly provided on the piston member of the syringe, and a biomolecular analysis section which performs biomolecular analysis by making the sensor probe pierce the living body tissue that is collected in the tissue-sampling section.

10. A living body tissue analyzing and processing system comprising:
tissue-sampling means for sucking and sampling a living body tissue; and
tissue-processing means for subjecting the living body tissue, which is collected by the tissue-sampling means, to a process for analysis.

11. The living body tissue analyzing and processing system according to claim 10, wherein the tissue-processing means is means for subjecting the living body tissue to at least one of freezing, freezing/drying, division, crushing, ethanol fixation and formalin fixation.

12. A living body tissue analyzing and processing system comprising:
an endoscope which observes an inside of a living body; and
an endoscopic therapeutic device which is inserted in the living body through a therapeutic device channel of the endoscope,
wherein the endoscopic therapeutic device comprises:
an elongated insertion section which is inserted into the living body through the therapeutic device channel;
a tubular puncture needle which is disposed at a distal end portion of the insertion section and is made to pierce a living body tissue;
tissue-sampling means for sampling the living body tissue in a state in which the puncture needle pierces the living body tissue; and
tissue-processing means for subjecting the living body tissue, which is collected by the tissue-sampling means, to a process for analysis.

13. The living body tissue analyzing and processing system according to claim 12, wherein the tissue-sampling means comprises a syringe which includes an outer cylinder member which is detachably coupled to a proximal end portion of the tube body, and a shaft-shaped piston member which is axially slidably inserted in a cylinder of the outer cylinder member, the living body tissue being collected into an inside of the outer cylinder member through the puncture needle and the tube body by a suction operation in which the piston member is slid in an outward direction of the outer cylinder member,
the tissue-processing means includes a living body tissue freezing mechanism which freezes the living body tissue that is collected in the syringe, and
the living body tissue freezing mechanism includes a Peltier element which is mounted in the outer cylinder member of the syringe, and a power supply device which supplies power to the Peltier element.

14. The living body tissue analyzing and processing system according to claim 12, wherein the tissue-sampling means comprises a syringe which includes an outer cylinder member that is detachably coupled to a proximal end portion of the tube body and a shaft-shaped piston member that is axially slidably inserted in a cylinder of the outer cylinder member, the living body tissue being collected into an inside of the outer cylinder member through the puncture needle and the tube body by a suction operation in which the piston member is slid in an outward direction of the outer cylinder member, and
the tissue-processing means includes a biomolecular analysis section which includes a treatment member that subjects the living body tissue, which is collected in the syringe, to a process for analysis, and performs biomolecular analysis of the living body tissue by putting the treatment member in contact with the living body tissue.

15. A sample-taking method for a tissue analysis process, comprising:
a step of inserting an endoscopic therapeutic device, which includes a tubular puncture needle, into a living body through a therapeutic device channel of an endoscope which observes an inside of the living body;
a step of making the puncture needle pierce a living body tissue;
a tissue-sampling step of sampling the living body tissue into the endoscopic therapeutic device in a state in which the puncture needle pierces the living body tissue;
a tissue freezing process step of subjecting the living body tissue, which is collected in the tissue-sampling step, to a freezing process for analysis; and
a step of taking out a pellet of a frozen piece of the living body tissue, which is subjected to the freezing process in the tissue freezing process step, from the endoscopic therapeutic device, and transferring the pellet to a tissue analysis process section in a later step.
